(19) Europäisches Patentamt
European Patent Office
Office européen des brevets

(11) **EP 1 156 333 B1**

(12) **EUROPEAN PATENT SPECIFICATION**

(45) Date of publication and mention
of the grant of the patent:
**01.08.2007 Bulletin 2007/31**

(51) Int Cl.:
***G01N 33/68*** *(2006.01)*

(21) Application number: **01303685.0**

(22) Date of filing: **23.04.2001**

(54) **A method of measuring the duration of adequate immune memory in companion animals**

Verfahren zur Messung der Dauer eines adäquaten Immungedächtnisses in Haustieren

Procédé pour mesurer la durée d'une mémoire immunitaire adéquate dans les animaux de compagnie

(84) Designated Contracting States:
**AT BE CH CY DE DK ES FI FR GB GR IE IT LI LU MC NL PT SE TR**

(30) Priority: **27.04.2000 US 200041 P**

(43) Date of publication of application:
**21.11.2001 Bulletin 2001/47**

(73) Proprietor: **Pfizer Products Inc.**
**Groton,**
**Connecticut 06340 (US)**

(72) Inventors:
• **Coyne, Michael Joseph**
**Oxford,**
**Pennsylvania 19363 (US)**
• **Kowalski, Dennis Joseph**
**South Salem,**
**New York 10590 (US)**
• **McGavin, David Ross,**
**Pfizer Global Res. and Dev.**
**Groton,**
**Connecticut 06340 (US)**
• **O'Hara, Michael Kenneth,**
**Pfizer Global Res. Dev.**
**Groton,**
**Connecticut 06340 (US)**

(74) Representative: **Williams, Aylsa**
**D Young & Co**
**120 Holborn**
**London EC1N 2DY (GB)**

(56) References cited:

• SCOTT F W ET AL: "Duration of immunity in cats vaccinated with an inactivated feline panleukopenia, herpesvirus, and calicivirus vaccine." FELINE PRACTICE, vol. 25, no. 4, 1997, pages 12-19, XP001024430 ISSN: 1057-6614
• SCOTT FRED W ET AL: "Long-term immunity in cats vaccinated with an inactivated trivalent vaccine." AMERICAN JOURNAL OF VETERINARY RESEARCH, vol. 60, no. 5, May 1999 (1999-05), pages 652-658, XP001024431 ISSN: 0002-9645
• OLSON P ET AL: "SERUM ANTIBODY RESPONSE TO CANINE PARVOVIRUS CANINE ADENOVIRUS-1 AND CANINE DISTEMPER VIRUS IN DOGS WITH KNOWN STATUS OF IMMUNIZATION STUDY OF DOGS IN SWEDEN" AMERICAN JOURNAL OF VETERINARY RESEARCH, vol. 49, no. 9, 1988, pages 1460-1466, XP001024400 ISSN: 0002-9645
• MCKINNEY W P ET AL: "DURATION OF RESPONSE TO INTRAMUSCULAR VERSUS LOW DOSE INTRADERMAL HEPATITIS B BOOSTER IMMUNIZATION" INFECTION CONTROL AND HOSPITAL EPIDEMIOLOGY, vol. 12, no. 4, 1991, pages 226-230, XP001024413 ISSN: 0899-823X
• LI VOLTI S ET AL: "Duration of hepatitis B antibody response in children immunised with hepatitis B and compulsory vaccines." EUROPEAN JOURNAL OF EPIDEMIOLOGY, vol. 11, no. 2, 1995, pages 217-219, XP001024410 ISSN: 0393-2990
• SIMONSEN O ET AL: "EVALUATION OF VACCINATION REQUIREMENTS TO SECURE CONTINUOUS ANTITOXIN IMMUNITY TO TETANUS" VACCINE, vol. 5, no. 2, 1987, pages 115-122, XP001024409 ISSN: 0264-410X

EP 1 156 333 B1

**Description**

Background of the Invention

The purpose of vaccination is two fold: to protect the individual from disease and to maintain a large enough number of immune individuals in the population so that the disease is not readily transmitted. As long as there is risk of infection, a threshold level of immune individuals is required to maintain herd immunity. If the number of immune individuals in the population decreases below this threshold, either due to waning of vaccine protection or non-vaccination, disease outbreaks can occur. Thus, an important parameter of a vaccine is how long it is able to protect the animal from infection.

Currently, because little to no duration of immunity information is available for most animal vaccines, annual vaccination is recommended by most vaccine labels and veterinarians (Schultz R.D. Veterinary Medicine 93:233-254 (1998)). These annual vaccine administration recommendations had been made as early as 1961 and have since been established as the standard practice by veterinary organizations. But recent information suggests that many vaccines provide effective immunity in companion animals for years; therefore, annual vaccine administration may not be necessary (Macy D.W. J. Am. Vet. Med. Assoc. 207:421-425 (1995)).

Until recently, most vaccines were evaluated for duration of immunity for only a few weeks or months but not necessarily the full vaccination interval touted on the label. Recently, USDA rules have been expanded to require that newly approved novel veterinary vaccines be shown to induce the minimum duration of immunity stated on the product label, but, manufacturers are not required to establish maximum duration of immunity provided by the vaccine (1998 Report of the American Association of Feline Practitioners and Academy of Feline Medicine Advisory Panel on Feline Vaccines J. Am. Vet. Med. Assoc. 212, 227-241 (1998).

In general, it is thought that the risks of side effects to animals is outweighed by the benefit imparted by annual vaccine administration even though most vaccines lack supportive experimental data documenting a need for annual vaccine administration (1998 Report of the American Association of Feline Practitioners and Academy of Feline Medicine Advisory Panel on Feline Vaccines J. Am. Vet. Med. Assoc. 212, 227-241 (1998)). But today the incidence of severe vaccination side effects in animals is more apparent and the risk/benefit ratio for annual vaccine administration no longer appears as favorable (Smith J. Am. Vet. Med. Assoc. 207, 421-425 (1995); Hendrick et al. Cancer Research 52, 5391-5394 (1992) ; Duval et al. Journal of Veterinary Internal Medicine 10, 290-295 (1996)). Adverse responses range from minor local reactions and stinging to severe and sometimes fatal anaphylaxis or development of neoplasia. In short, an animal does not benefit and may be placed at serious risk when vaccinated unnecessarily (W. Jean Dodds In Advances in Veterinary Medicine, Vol. 41, Veterinary Vaccines and Diagnostics, Ronald D. Schultz ed. p. 718-719 (1999)). Nevertheless, the companion animal community has - since sufficient information on duration of immunity is lacking - reconciled the risk of over-vaccination with the benefit provided by vaccines in preventing serious disease in animals. But this argument becomes less forceful in light of newer vaccines for diseases that either don't occur with high frequency in unvaccinated animals or that are that are less serious (e.g., Lyme disease, feline leukemia, feline infectious peritonitis, and ringworm).

Conventionally, duration of immunity has been established by selecting an appropriate number of immunologically naive animals (*e.g.*, 20-100), vaccinating with a minimum immunizing dose of the experimental vaccine or placebo, holding the animals in strict isolation for the desired period, and then challenging with the virulent organism. The clinical disease observed in vaccinates is compared to that of non-vaccinates. A disadvantage of this approach is that it is not "real world". That is, the challenge is typically made neither with currently circulating strains of the infectious agent in question nor by the route or means inherent in the field setting. Furthermore, these studies, out of necessity, are long, labor intensive, and costly, as well as often destructive to the animals. It should also be recognized that the longer the study period, the greater the risk that the study animals may inadvertently be exposed to the pathogen that is the subject of the study, thereby invalidating the study. These factors are major considerations when pharmaceutical companies set up duration of immunity studies.

In general, the response of an animal's immune system to infection or immunization can be divided into two functional categories: humoral immunity and cellular immunity. Humoral immunity involves plasma cells (activated B cells) and memory B cells. During a humoral immune response, antigen specific antibodies generated by plasma cells neutralize or opsonize extracellular infectious agents and/or virulence factors. Humoral immunity is important for protection against extracellular phases of systemic viral and bacterial infection.

Cellular immunity involves the production of effector T-cells, memory T-cells (CD4*T and CD8$^+$T) and NK cells that are responsible for the direct (cell-to-cell) or indirect (via cytokines) destruction of infected cells (Wood PR et al. Veterinary Immunology & Immunopathology 54:33-44 (1996) and Allen JE et al. Immunology Today 18:387-392 (1997)). Cellular immunity is important in fighting intracellular pathogens.

In addition to humoral and cellular immunity, the non-specific immune system (*i.e.,* phagocytic cells) aids in initial control of infection but is not affected by vaccination or prior exposure.

The immune system self-regulates between a humoral and cellular immune response so that following infection or immunization, one predominates. The type of response that predominates is related to the type of infectious agent, the route of infection, the pathogenesis of the agent (local versus systemic), and in the case of inactivated vaccines, the inactivating agent and type of adjuvant used.

It is evident that some plasma cells must persist for long periods of time, since antibody titers can persist in the blood for years after infection or vaccination without re-exposure. The presence of these pre-existing systemic neutralizing or opsonizing antibodies at the site of infection is effective in preventing infection and providing protective immunity for many years (Plotkin SA & Mortimer EA. Eds., 1994. Vaccines. Second Edition, WE Saunders, Philadelphia).

Assay-based methods (rather than pathogenic challenge with the virulent infectious agent) may be used as an indication of the presence of adequate immune memory or protective immunity (Olson et al. Am. J. Vet. Res. 49, 1460-1466 (1988), Sprent et al. Science 265, 1395-1400 (1994), Alderink et al. Am. J. Vet. Res. 207, 1016-1018 (1995) Schultz (1995), Smith Am. J. Vet. Res. 207, 421-425 (1995)). For many diseases, an adequate immune memory can be verified by detecting a rapid immune system response (*i.e.*, an anamnestic response) induced by exposure to a specific antigen, for example, detection of antibodies in serum or serologic quantification of antibody formed in response to vaccine administration (Alderink et al. J. Am. Vet. Med. Assoc. 207, 1016-1018 (1995); Dodds Proc. Am. Holistic Vet. Med. Assoc. 74-80 (1995); Dodds Vaccine Related issues In Complementary and Alternative Veterinary Medicine, A.M. Schoen and S.G. Wynn, eds. pp. 701-702 (1997); Schultz (1995); Scott et al. Feline Pract. 25, 12-19 (1997)).

But immunity against infectious agents for which a cellular response is the primary mode of protection may not correlate with antibody levels. In these cases, an adequate immune memory may be determined by a cellular immunoassay. For example, with feline rhinotracheitis virus (Johnson et al. Journal of the American Veterinary Medical Association 186, 149-152 (1985)) and feline infectious peritonitis virus (Pedersen Feline Practice 23, 7-20 (1995)), which are counteracted in companion animals primarily by a cellular immune response, there is no correlation of antibody level with protection. A higher correlation exists with antibody levels for protection against feline leukemia virus (Haffer et al. Vaccine 8,12-16 (1990)), where both cellular and humoral immunity are involved. And a strong correlation exists where humoral immunity alone is required for protection, for example, against *Leptospira canicola* (Hartman et al. Veterinary Immunology and Immunopathology 7, 245-254 (1984)).

Other factors should be considered when choosing an assay-based procedure to determine an immunological memory. For example, with diseases that replicate and cause damage primarily on mucosal surfaces - *e.g.*, *Bordetella bronchiseptica,* canine coronavirus, canine parainfluenza, and *Chlamydia psittaci* - serological data may not correlate extensively with protection (Tennant et al. Research in Veterinary Science 51, 11-18 (1991); Bey et al. American Journal of Veterinary Research 42, 1130-1132 (1981); and Mitzel et al. American Journal of Veterinary Research 38, 1361-1363 (1977)). This is because most of the antibody classes responsible for systemic humoral immunity, as well as the white blood cells responsible for cell mediated immunity, are not present on the mucosal surface and a blood serum based immune response is generally not rapid enough to provide protective antibody or white blood cell levels to the mucosal layer. A serum based immune response will, however, in most cases lessen the severity of mucosally based diseases.

While serological data are useful to provide evidence of an immunological memory against a particular pathogen, quantitative evaluation is generally not informative because the assay methodologies and titer levels have not been standardized between assay methods or laboratories (Luff et al. Vet. Rec. 120, 270-273 (1987)). Not only will the use of different assays cause variance, but, even when the same assays are used, the numerical values will depend on the technician's skill, technique, and equipment.

In conclusion, while methods exist to determine whether an animal has a marker of immunity, no practical methodology exists to determine the duration of immunity for animal vaccines. This objective is now met by the method of the present invention as described below.

Summary of the Invention

The present invention provides efficient methodology to measure the duration of adequate immune memory for animal vaccines. The invention involves a retrospective analysis utilizing veterinary vaccine administration histories and clinical histories, and markers of immunity of animals in the field (rather than in the laboratory), to derive a vaccine's duration of adequate immune memory. The vaccine may be any vaccine known or to be developed and may be a single component vaccine or may also comprise more than one immunogenic component.

According to the current invention, since challenge occurs in the field under natural conditions, environmental (temperature, humidity, indoor/outdoor), physiological (nutrition, exercise) and psychological (stress of lifestyle or warmth of family setting) variables inherent in the field setting are incorporated in vaccine assessment. This is in contrast to traditional challenge studies using a single laboratory strain of the infectious agent, which may or may not be current with respect to the field epidemiological situation. Thus, in the traditional hold and challenge in confinement approach, these variables are less likely to be representative of the real-world situation and hence, the laboratory measured duration of immunity may not correspond with a field duration of immunity.

In one embodiment the invention concerns a method of determining a duration of adequate immune memory induced by a vaccine, for a disease, in an animal where the animal is a dog or a cat, the method comprising:

(a) selecting a plurality of study animals from one or more clinics,

i) where each animal has been vaccinated with the vaccine,
ii) where a time since a last vaccination date is at least about one year
iii) where the animal has been living in a field environment for at least about one year after the last vaccination date and
iv) where each animal has a vaccine and health record;
v) where each animal has been evaluated for risk of exposure to said disease;

(b) assign said animals a risk category according to the following:

i) animals are considered high risk or low risk based on exposure to other animals or disease vectors

(1) low risk animals are kept indoors and or are NOT exposed to other animals and disease vectors,
(2) high risk animals are animals not kept indoors and ARE exposed to other animals, or disease vectors,

(c) assigning each animal an indicator of adequate immune memory, where that indicator is either as a non-immune animal, aka first indicator animal, or an immune animal, aka second indicator animal comprising:

i) evaluate said animals for clinical signs of said disease using observations and said health records since last vaccination date,
ii) identify evaluated animals having signs of said disease, these are non-immune animals, aka first indicator animals,
iii) identify said high risk animals living in areas of disease prevalence and not having signs of said disease determined by clinic records and/or lab confirmation of disease cases in said living area, these are immune animals, aka second indicator animals,
iv) evaluate a blood serum sample from each animal that has not shown clinical signs of the disease and that is not high risk animal living in an area of disease prevalence and not having signs of said disease since the last vaccination date,
v) identify those animals having either a cellular immune response and or an antibody titer of at least 2, these are immune animals, aka second indicator animals,
vi) identify those animals having no cellular immune response and or an antibody titer less than 2,
vii) administer a booster dose of the vaccine to these animals
viii) evaluate a blood serum sample from each animal that has received the booster dose 3 days to 28 days following the booster dose
ix) identify those animals having an adequate anamnestic response of at least a 4-fold increase in serum antibody titer, or a statistically significant increase in cellular immune response, these are immune animals, aka second indicator animals,
x) identify those animals not having an adequate anamnestic response of a 4-fold increase in serum antibody titer, or a statistically significant increase in cellular immune response, these are non-immune animals, aka first indicator animals, and

(d) determining the duration of adequate immune memory for said animals, by determining this from a duration of adequate immune memory estimation equation, said duration of adequate immune memory estimation equation derived by a logistic regression analysis of the first and the second indicator and the vaccine administration record.

In an embodiment, the method further comprises: (a) assigning each animal that has displayed clinical signs of the disease since the last vaccination or that neither displays an adequate antibody titer, nor an adequate cellular titer nor an adequate anamnestic response the first indicator, (b) designating each animal which is not assigned the first indicator as either a high risk animal or a low risk animal; (c) assigning each low risk animal that displays either a cellular immune response or that displays either the adequate antibody titer or the sufficient anamnestic response the second indicator, (d) assigning each high risk animal that has no history of the disease in question and where there is evidence of prevalence of the disease in question in the region the second indicator; and (e) assigning each high risk animal that has no history of the disease in question that displays either the cellular immune response, or the adequate antibody titer or the sufficient anamnestic response, the second indicator.
In still another preferred embodiment, the step of determining the duration of adequate immune memory from the first and the second indicators and the vaccine administration record comprises:

(a) determining an enrolment date for each animal, the enrolment date being when evaluation of the animal to detect the marker of immunity was begun;
(b) assigning a start of study date as the enrolment date for a first animal of the plurality of animals enrolled in the study;
(c) assigning a variable $X_j$ for each animal as a number of days between the start of study date and the enrolment date for the animal
(d) assigning a variable $X_j$ for each animal as a number of days between the last vaccination date for the animal and the enrollment date for the animal.
(e) determining the duration of adequate immune memory from a duration of adequate immune memory estimation equation, said duration of adequate immune memory estimation equation derived by a logistic regression analysis of the first and second indicators and the variables $X_i$ and $X_1$.

In an embodiment, the duration of adequate immune memory estimation equation is in a form $logit(E) = \beta_0 + \beta_1 X_{01} + \beta_2 X_{CV} + C_k$ where:

E is a desired level of efficacy;
$X_{DI}$ is the duration of adequate immune memory;
$X_{CV}$ is a mean of $X_1$;
$C_k$ is a constant representing a random effect to account for variation between the clinics and is derived by logistic regression; and
$\beta_0, \beta_1, \beta_2$ are constants derived by logistic regression.

In a more preferred embodiment thereof, the model for the logistic regression to derive the values of $C_k, \beta_0, \beta_1,$ and $\beta_2$ is in a form $logit(p) = \beta_0 + \beta_1 X_1 + \beta_2 X_1 + C_k$, where:

$logit(p)$ is a vector representing the immune statuses for the animals; and
$C_k$ is the clinic from which each animal was selected.

These and other features, aspects, and advantages of the invention will become better understood with reference to the following detailed description, examples, and appended claims.

Detailed Description of the Invention

As used herein, the term "duration of adequate immune memory" for a particular vaccine means the estimated time that an animal, vaccinated with the vaccine, will display a marker of immunity to the disease for which the vaccine is prescribed.
As used herein, the term "marker of immunity" means any indication that an animal-which has not shown clinical signs for the disease in question since the last vaccination date-has an immunologic memory to the disease. According to the definition of "marker of immunity", an animal that has shown clinical signs of the disease in question since the last vaccination date does not have a marker of immunity for the disease. Examples of markers of immunity include those detected by serologic assays, such as circulating antibody titers, secretory antibody titers, cellular responses (*e.g.*, B or T-cell activity), cytokine levels (*e.g.*, tumor necrosis factor, gamma interferon, *etc.*) and other assays known to those skilled in the art. Other markers are detected by non-serologic analysis, for example, if, in high exposure risk area (*e.g.*, high disease prevalence or high population density), the animal does not display the disease's symptoms. This is particularly useful when the disease symptoms are unique. Suitable markers of immunity vary widely with the disease and the animal's exposure risk. One skilled can readily choose the appropriate marker of immunity depending on the disease, the animal, and the risk factors.
As used herein, the term "animal" means any animal that can benefit by vaccination, including, but not limited to, companion animals; livestock and farm animals, such as cows, sheep, goats, and swine; fowl, such as chickens, turkeys, and ducks, and wildlife such as deer and fish. Preferably, the animal is a companion animal.
As used herein the term "companion animal" is any tamed or domesticated animal that is kept for human companionship, for example, cats, dogs, horses, birds, snakes, ferrets, hamsters, and the like.
As used herein, a "field environment" means a non-laboratory setting where the animal lives under the typical conditions and setting for which the animal is adapted or bred. For example, the field environment for a companion animal is the typical conditions and setting of the pet/owner relationship, such as the owner's household, yard, or barn.
As used herein, the term clinic" means a particular sampling pool of animals. For example, a clinic for a particular study may be defined as a particular geographical area. One of skill in the art will readily know how to define the clinic for a duration of adequate immune memory study depending on the specific disease and animal under evaluation. Examples of clinics include but are not limited to veterinary hospitals, feed lots, farms, and barns. The number, scope, and selection of clinics for a particular study will vary with the nature and prevalence of the disease and one skilled in the art will know how to select such variables to ensure validity of the study.

The invention is of a general nature and useful to establish the duration of adequate immune memory induced by any vaccine, known or to be discovered, in any animal. For example, suitable animals include but are not limited to, farm animals such as, bovine, equine, swine, poultry, as well as wildlife, such as fish and birds. The invention is particularly suited to measure a vaccine's duration of adequate immune memory in companion animals such as dog or cats. The vaccine type may be any of those known in the art or to be developed, for example, modified live vaccines, killed vaccines, DNA vaccines, vectored vaccines, or subunit vaccines or any other vaccine type known or to be discovered. Examples of cat and dog vaccines include both core and non-core vaccines. Examples of feline core vaccines include vaccines effective for panleukopenia, feline viral rhinotracheitis, calicivirus and rabies, while feline non-core vaccines include vaccines for infectious peritonitis, leukemia, and *Chlamydia psittaci*. Canine core vaccines include vaccines designed for distemper, parvovirus, adenovirus, and rabies, while canine non-core vaccines include vaccines against parainfluenza, Bordetella, Borrellia, and Leptospira.

The vaccine administration method may be any method known in the art such as, parenteral or intranasal. In one embodiment, the invention provides a method of determining a duration of adequate immune memory induced by a vaccine, for a disease, in an animal where the animal is a dog or a cat, the method comprising:

(a) selecting a plurality of study animals from one or more clinics,

   i) where each animal has been vaccinated with the vaccine,
   ii) where a time since a last vaccination date is at least about one year
   iii) where the animal has been living in a field environment for at least about one year after the last vaccination date and
   iv) where each animal has a vaccine and health record;
   v) where each animal has been evaluated for risk of exposure to said disease;

(b) assign said animals a risk category according to the following:

   i) animals are considered high risk or low risk based on exposure to other animals or disease vectors

      (1) low risk animals are kept indoors and or are NOT exposed to other animals and disease vectors,
      (2) high risk animals are animals not kept indoors and ARE exposed to other animals, or disease vectors,

(c) assigning each animal an indicator of adequate immune memory, where that indicator is either as a non-immune animal, aka first indicator animal, or an immune animal, aka second indicator animal comprising:

   i) evaluate said animals for clinical signs of said disease using observations and said health records since last vaccination date,
   ii) identify evaluated animals having signs of said disease, these are non-immune animals, aka first indicator animals,
   iii) identify said high risk animals living in areas of disease prevalence and not having signs of said disease determined by clinic records and/or lab confirmation of disease cases in said living area, these are immune animals, aka second indicator animals
   iv) evaluate a blood serum sample from each animal that has not shown clinical signs of the disease and that is not high risk animal living in an area of disease prevalence and not having signs of said disease since the last vaccination date,
   v) identify those animals having either a cellular immune response and or an antibody titer of at least 2, these are immune animals, aka second indicator animals,
   vi) identify those animals having no cellular immune response and or an antibody titer less than 2,
   vii) administer a booster dose of the vaccine to these animals
   viii) evaluate a blood serum sample from each animal that has received the booster dose, 3 days to 28 days following the booster dose, identify those animals having an adequate anamnestic response of at least a 4-fold increase in serum antibody titer, or a statistically significant increase in cellular immune response, these are immune animals, aka second indicator animals,
   ix) identify those animals not having an adequate anamnestic response of a 4-fold increase in serum antibody titer, or a statistically significant increase in cellular immune response, these are non-immune animals, aka first indicator animals, and

(d) determining the duration of adequate immune memory for said animals, by determining this from a duration of adequate immune memory estimation equation, said duration of adequate immune memory estimation equation derived by a logistic regression analysis of the first and the second indicators and the vaccine administration record.

The number of animals employed in the study should be statistically significant. One skilled in the art can readily determine a statically significant group of animals depending on the area's disease prevalence, population density, and the disease under study, using well known techniques (Steel, R.G.D. and Torrie, J.H. (1980), Principles and Procedures of Statistics: A Biometrical Approach. 2nd Edition; and Cannon, R. M. and Roe, R.T. (1982) Livestock Disease Surveys: A Field Manual for Veterinarians, both of which are incorporated herein by reference).

Preferably, at least about 100 animals are included in the study, more preferably about 200 to about 10,000 animals, most preferably about 300 to 1000 animals. In general, the larger the number the more accurate the study, although the difficulty in selecting the animals and collecting the data rises proportionally.

Preferably, each study animal was vaccinated with the same vaccine (*i.e.*, prepared by the same manufacturer by the same method), by the same vaccine administration route (*e.g.*, injection, subcutaneous, parenteral, intramuscular, intranasal), at the same vaccination site. Preferably, animals to which the vaccine was improperly administered (*e.g.*, the instructions on the label were not followed or an expired vaccine was administered, *etc.*) are excluded from the study group.

The plurality of study animals that comprise the study group may be selected from clinics of different risk and geographical locations. One skilled in the art will readily be able to choose the study group according to the vaccine, disease, animal, marker of immunity to be evaluated, and the animal's exposure risk. When, because of the nature of the study, the animals are selected from a high exposure risk area, the preferred marker of immunity is lack of clinical signs for the disease. Preferably, such disease signs would be pathognomonic. On the other hand, when the animals are selected from a low exposure risk area, the preferred marker of immunity is evidence of a vaccine response (*e.g.*, serologic assay). These markers are preferred because when the study animals are selected from a high risk area, actual disease exposure may be the origin of a serologic immune memory rather than the vaccine, thus serologic data are inconclusive. And, of course, when the study animals are selected from a low exposure risk area, the absence of clinical signs is not a useful marker of immunity.

Factors associates with disease exposure risk include the animal's environment *(inter alia,* the prevalence of the disease and animal population density in the animal's particular geographic location, the number of like animals in the household (when the animal is a companion animal), whether the animal is an indoor or outdoor animal, whether the animal is periodically housed in boarding houses or kennels, and if the animal has occasion to roam free), age, general health, current medications, sex, and any disease that the animal has contracted.

An animal's exposure risk to a specific disease in a particular geographical area is readily determined by one skilled in the art. Risk factors may be estimated by various means. Such methods of disease risk estimation may include published data, epidermiotogical modelling using published factors, regional/local Diagnostic Laboratory sample results (i.e., most labs keep records of submissions and diagnosis, enabling a calculation of % positive) and clinic surveys.

In a preferred embodiment of the invention, the minimum age for animals is 6 to 8 weeks. This because high levels of maternal antibodies in young animals (under about 6 to 8 weeks of age) can prevent some vaccines from being effective. An animal's immune system responds most effectively to vaccination after decay or disappearance of the maternal anitibodies (Schultz Vet. Med. 223-254, March 1998 and Serge Martinod In Advances in Veterinary Medicine, Vol. 41, Veterinary Vaccines and Diagnostics, Ronald D. Schultz ed. p. 661 (1999)).

The animal's vaccine administration and clinical history are obtained to determine the last vaccination date, whether the animal has been living in a field environment for about a year after the last vaccination date, and whether the animal has manifested clinical signs of the disease since the last vaccination date. Preferably the animal's vaccine administration and clinical history also includes the vaccine's route of administration and administration site, manufacturer, lot and serial number, and expiration date. Even more preferably, the animal's history further includes factors that indicate the animal's risk of contracting the disease, for example, the animal's environment as well as the animal's age, general health, current medications, sex, breed, whether the animal has been neutered or spayed, and any diseases that the animal has contracted. Data concerning the animal's environment preferably includes such factors as the prevalence of the disease in the animal's particular geographic location, population density of the animal under study, the number and type of like animals in the household, whether the animal is an indoor or outdoor animal, whether the animal is periodically housed in boarding houses or kennels, and whether the animal has occasion to roam free. Of course, the higher the animal's incidence of contact with a large number of other like animals, the higher the animals exposure risk. Poor general health, old age, or other conditions that compromise the immune system will also put an animal at a higher risk of contracting the disease in question. Furthermore, past incidence of disease and current prescription medications may also put the animal at a higher risk (1998 Report of the American Association of Feline Practitioners and Academy of Feline Medicine Advisory Panel on Feline Vaccines J. Am. Vet. Med. Assoc. 212, 227-241 (1998).

The animal's vaccine administration and clinical history are available from veterinary and animal owner records. Currently no prescribed system of record keeping exists among veterinarians, but it is recommended by the companion animal community that record keeping be meticulous and methodical (Report of the American Association of Feline Practitioners and Academy of Feline Medicine Advisory Panel on Feline Vaccines J. Am. Vet. Med. Assoc. 212, 227-241 (1998)). One method to facilitate transfer of veterinary and companion animal owner records to the manufacturer conducting a duration of adequate immune memory study is by supplying a prestamped questionnaire to the veterinarian for compiling the animal's clinical and vaccine administration history. The questionnaire may have a preferred time of submission, after which, the companion animal owner and the veterinarian may complete and submit it to the manufacturer. Peel off vaccine labels to be affixed to the questionnaire can facilitate such record keeping.

Once the study group has been selected and the clinical and vaccine administration histories examined and compiled, the immune status for each study animal is assigned. A first indicator is assigned to each animal that does not display a marker of immunity, preferably the first indicator is a 0. And a second indicator is assigned to each animal that displays a marker of immunity, preferably the second indicator is a 1.

The particular marker of immunity assayed for will depend upon the particular disease for which the vaccine is being tested. For the purposes of the present invention, each animal that has manifested clinical signs of the disease under study, since the last vaccination date does not have a "marker of immunity". Markers of immunity and methods for their assay are well known in the art, and the ordinary artisan will know which procedure to select depending on the study. For example, a blood serum sample, obtained from the animal's owner or veterinarian, may be analyzed for the presence of circulating antibodies. Another marker of immunity is an anamnestic response to the disease in question i (Manual of Clinical Laboratory Immunology, Fifth Edition, Editor: Noel R. Rose et al. (1997); Manual of Clinical Microbiology, 7th Edition, Editor: Patrick R. Murray et al. (1999); Clinical Microbiology Procedures Handbook, Editor: Henry D. Isenberg et al. (1992); S.Faine, Leptospira and Leptospirosis, Second Edition, (1994)).

Where the primary immune response for a specific disease is cell-mediated, there are a variety of assays that can be used to identify animals with a marker of immunity. For example see, (Manual of Clinical Laboratory Immunology, Fifth Edition, Editor: Noel R. Rose et al. (1997)). Table 1 below list a few examples of suitable markers of immunity for a variety of vaccines.

TABLE 1: Markers of immunity for various canine vaccines

| Disease for which the vaccine is prescribed | Marker | References |
|---|---|---|
| Rabies | Seroneutralizing titers | World Health Organization. 1992. Expert Committee on Rabies, 8th Report. World Health Organization, Technical Report Series no. 824, Geneva. |
| Canine Distemper | Seroneutralizing titers Ab | Cooper et al. Bulletin Mensuel de la Societe Veterinaire Pratique de France 75,13 1-152 (1991). Auby et al. Recueil de Médecine Vétéinaire 150, 33-36 (1974). |
| Canine Parvovirus | Seroneutralizing titers Ab | Pollock et al. Journal of the American Veterinary Medical Association 180, 37-43 (1979). |
| Canine Adenovirus CAV-1 | Seroneutralizing titers Ab | Hartman et al. Veterinary Immunology and Immunopathology 7, 245-254 (1984). |
| *Leptospira canicola* and *icterohaemorrhagiae* | Neutralizing IgG titers | Hartman et al. Veterinary Immunology and Immunopathology 7. 245-254 (1984). |
| Canine Coronavirus | Secretory IgA | Tennant et al. Research in Veterinary Science 51, 11-18 (1991). |

(continued)

| Disease for which the vaccine is prescribed | Marker | References |
|---|---|---|
| Bordetella BronchisepticalCanine Parainfluenza | Secretory IgA | Bemis DA. 1976. Bordetella bronchiseptica in dogs: Bacteriological studies, pathogenesis, immune response and antibiotic treatment. Ph.D. Thesis. Cornell University |
| Panleukopenia | Seroneutralizing titers Ab | Scott et al. Journal of the American Veterinary Medical Association 156, 439-453 (1970). Johnson et al. Journal of the American Veterinary Medical Association 158, 876-884 (1971). |
| Rhinotracheitis | Role of cell mediated immunity | Johnson et al. Journal of the American Veterinary Medical Association 186, 149-152 (1985). |
| Rabies | Seroneutralizing titers | World Health Organization. 1992. Expert Committee on Rabies, 8th Report. World Health Organization, Technical Report Series no. 824, Geneva. |
| Calicivirus | Seroneutralizing titers Ab | Johnson et al. Journal of the American Veterinary Medical Association 186, 149-152 (1985). - |
| *Chlamydia psittaci* | Secretory IgA | Mitzel et al. American Journal of Veterinary Research 38,1361-1363 (1977). |
| FeLV | Seroneutralizing titers Ab | Haffer et al. Vaccine 8:12-16 (1990). Tompkins et al. Companion Animal Practice July: 15-26 (1988). |
| infectious Peritonitis | Salivary IgA | Pedersen Feline Practice 23:7-20 (1995). |

In a preferred embodiment of the invention, a marker of immunity is established when the animal's blood serum contains either a blood serum antibody titer level of at least about 2 or if the animal displays a strong anamnestic response upon administration of a booster dose of the vaccine. A strong anamnestic response is a 4-fold increase in serum antibody titer when measured 3 days to 28 days following administration of a booster dose of the vaccine in question. The conventional practice is to draw a blood serum sample then administer the booster dose. Then, within 3 days to 28 days, a second blood serum sample is taken. Sera from the first and second blood samples are tested for antibody in parallel. In the presence of immunological memory in a healthy individual, antibody levels in the second serum sample will be 4-fold greater than levels in the first sample.

Once the immune status for each animal is established as described above, the data for the study group will be compiled and analyzed to determine the duration of adequate immune memory. Each animal's clinic ($C_k$), enrollment date, and last vaccination date will be recorded, and a value in days will be determined for each of $X_i$ and $X_j$, where:

$X_i$ is the $i^{th}$ animal's time in days between the animal's last vaccination date for the disease and the animal's enrollment date;
$X_j$ is the $j^{th}$ animal's time in days between the animal's enrollment date and the start of study date.

As used herein, the "enrollment date" for each animal is the day on which the animal was evaluated for a marker of immunity. If the animal is assayed for more than one marker of immunity, the enrollment date is the date on which the animal was evaluated for the first marker of immunity. For example, when the marker of immunity is detection of antibody titer levels, the enrollment date is the day on which the first blood sample is drawn for antibody titer analysis. If the antibody titer analysis is negative, and the animal is evaluated for an alternate marker of immunity, such as an anamnestic response, the enrollment date is still the day on which the first blood sample is drawn for antibody titer analysis.

Herein, when referring to the "last vaccination date", the "vaccination date" means the day on which the prescribed vaccine administration protocol is concluded. Note that this date will not necessarily correspond to the date a particular vaccine dose is administered. Often, companion animal vaccination involves a series of vaccine administrations at short time intervals to establish immunity. For example, it may be necessary to give two or more doses of a killed vaccine within a short time period to attain immunity, whereas, in the same animal, modified live vaccines often immunize after only one administration (Report of the American Association of Feline Practitioners and Academy of Feline Medicine Advisory Panel on Feline Vaccines J. Am. Vet. Med. Assoc. 212, 227-241 (1998). Another example where vaccination date must be differentiated from vaccine administration date is where young animals (less than about 8 to 16 weeks) are administered multiple vaccine doses, spaced at 2 to 6 week intervals. This vaccine administration protocol is prescribed because maternally inherited antibodies may be present in the animals blood serum. Maternally inherited antibodies can negate vaccination and since it is not known exactly when these antibodies will dissipate, multiple vaccine administrations are recommended. For most companion animals, maternally inherited antibodies disappear after about 16 weeks of age. For example, the recommended vaccine administration schedule for canine distemper in puppies is a series of three parenteral injections of a modified live vaccine (*i.e.*, at 6 to 8 weeks, at 10 to 12 weeks, and at 14-16 weeks) (Council Report Journal of the American Veterinary Medical Association 195, 314-317 (1989)). Accordingly, in this case, the vaccination date is the date on which the 14-16 week vaccine injection is administered. In the current invention, in cases where the prescribed vaccine administration protocol is not followed for a particular animal, it is preferable that the animal be omitted from the study.

The vaccine's duration of adequate immune memory is estimated by a duration of adequate immune memory prediction equation, appropriate for the statistical model. The prediction equation may be derived by logistic regression analysis of the immunity data obtained from the animals enrolled in the study. Derivation of prediction equations by logistic regression analysis of raw data is well known to those of skill in the art. Exemplary models can be found in the following references: Agresti (1990), Categorical Data Analysis, 84-91; Beitler et al. Biometrics, 41, 991-1000 (1985); Davidian et al. Biometrika 80, 475-488 (1993); Harville et al. Biometrics 40, 393-408 (1984); Hosmer et al. (1989), Applied Logistic Regression; Longford Computational Statistics and Data Analysis 17, 1-15 (1994).

Once the prediction equation for a particular vaccine is derived, it may be solved to obtain the duration of adequate immune memory for the vaccine under study by imputing a desired efficacy level. The desired efficacy level is the percentage of vaccinated animals that would not manifest clinical signs of the disease if a statistically significant group was exposed to the disease in question. Or, in other words, the efficacy level is the probability that a particular animal will show clinical manifestations if exposed to a virulent challenge of the disease. Efficacy is a relative term and no vaccine will induce complete immunity to all animals in the field. To be federally licensed, the vaccinated group must show significantly less disease than the non-vaccinated control group upon a virulent challenge. For most diseases, for USDA approval, the efficacy level must be 80% (*i.e.*, 80% of the vaccinates must not show illness, Serge Martinod In Advances in Veterinary Medicine, Vol. 41, Veterinary Vaccines and Diagnostics, Ronald D. Schultz ed. p. 635 (1999)).

The preferred prediction equation is:

$$\text{logit}(E) = \beta_0 + \beta_1 X_{DI} + \beta_2 X_{CV} + C_k$$

where:

E is the desired level of efficacy;
$X_{DI}$ is the duration of adequate immune memory;
$X_{CV}$ is the mean $X_j$ value obtained using a means averaging program such as the MEANS procedure of SAS [this value represents a covariate to adjust for possible seasonal differences];
$C_k$ is a constant representing the random effect in the model to account for the possible variation between clinics, Ck is derived by logistic regression; and
$\beta_0$, $\beta_1$. $\beta_2$ are constants derived by logistic regression.

The preferred statistical model for the logistic regression to derive the values of Ck, $\beta_0$, $\beta_1$, and $\beta_2$ is:

$$\text{logit}(p) = \beta_0 + \beta_1 X_i + \beta_2 X_j + C_k$$

where $X_i$, and $X_j$, are defined above, $C_k$ is the $k^{th}$ clinic (*i.e.*, the clinic corresponding to the particular animal), and logit (p) is a vector representing the immune status indicators for each animal in the study group (*e.g.*, a vector consisting of 0s and 1s). Thus p becomes the probability that a animal in the study will display a marker of immunity.

Any suitable statistical program may be used for the logistic regression. Example of suitable models may be found in the SAS software package (*e.g.*, Version 8), well known to those in the art. The NLMIXED procedure in SAS is preferred logistic regression program. But the LOGISTIC, TLOGISTIC, GENMOD or CATMOD procedures in the SAS package may also be used.

<u>Protocols for Determining Markers of Immunity</u>

<u>Serological analysis of canine distemper virus</u>

Norden Laboratories Dog Kidney cells of passage range 126-143 are plated at 16,000 cells per plate well in Eagles minimal essential medium/10% Fetal bovine serum. The plates are incubated at 37°C, 5% $CO_2$ in a humidified environment for 24hrs until the desired confluency of 20-30% is achieved. Heat inactivated canine serum samples, positive control and negative control sera are serially diluted 2-fold to appropriate levels in Eagles minimal essential medium. Each test well of the diluted serum is combined with an equal volume of virus suspension containing 100-316 tissue culture infectious dose 50% ($TCID_{50}$) of canine distemper virus. Typically, one can use a non-cytopathic virus. The serum/virus mixture is incubated for an hour to allow neutralization of virus by specific antibody. Challenge virus is titrated to verify the amount of virus used in the assay (back- titration). Following incubation, the fluid contents of the plates are removed and the plates are inoculated with the serum/virus mixture, four replicates per serum dilution. Plates are incubated for $6\pm1$ days at 37°C, 5% CO2 in a humidified environment. The plates are then fixed with 80% acetone, rinsed and stained with direct fluorescein isothiocyanate labeled anti-CDV polyclonal antibody. Following the staining procedure, the plates are read for the presence or absence of viral specific fluorescence. Wells exhibiting no fluorescence ("FA") are scored as a "+" denoting the presence of neutralizing antibody. Wells containing FA are scored as a "-" denoting the lack of neutralizing antibody. Serum neutralizing titer is calculated by Spearman-Karber. An assay must fulfill the following criteria to be considered valid: 1. The back-titer must be within 100-316 $TCID_{50}$ 2. The positive control serum must be within the established range (typically, 36-145). 3. The negative control serum must have a titer of $\leq2$.

<u>Serological analysis of canine adenovirus type 2</u>

Norden Laboratories Dog Kidney cells of passage range 126-143 are plated at 16,000 cells per plate well in Eagles minimal essential medium/10% Fetal bovine serum. The plates are incubated at 37°C, 5% $CO_2$ in a humidified environment for 3-5 days until the desired confluency of 85-95% is achieved. Heat inactivated canine serum samples, positive control and negative control sera are serially diluted 2-fold to appropriate levels in Eagles minimal essential medium. Each test well of the diluted serum is combined with an equal volume of virus suspension containing 100-316 tissue culture infectious dose 50% ($TCID_{50}$) of canine adenovirus type 2. Typically, one can use a non-cytopathic virus. The serum/virus mixture is incubated for an hour to allow neutralization of virus by specific antibody. Challenge virus is titrated to verify the amount of virus used in the assay (back-titration). Following incubation, the fluid contents of the plates are removed and the plates are inoculated with the serum/virus mixture, four replicates per serum dilution. Plates are incubated for 7+1 days at 37°C, 5% CO2 in a humidified environment. The plates are then fixed with 80% acetone, rinsed and stained with direct fluorescein isothiocyanate labeled anti-CAV polyclonal antibody. Following the staining procedure, the plates are read for the presence or absence of viral specific fluorescence. Wells exhibiting no FA are scored as a "+" denoting the presence of neutralizing antibody. Wells containing FA are scored as a "-" denoting the lack of neutralizing antibody. Serum neutralizing titer is calculated by Spearman-Karber. An assay must fulfill the following criteria to be considered valid: 1) The back-titer must be within 100-316 $TCID_{50}$ 2) The positive control serum must be within the established range (typically, 25-99). 3) The negative control serum must have a titer of $\leq2$.

<u>Serological analysis of canine parainfluenza virus</u>

Norden Laboratories Dog Kidney cells of passage range 126-143 are plated at 16,000 cells per plate well in Eagles minimal essential medium/10% Fetal bovine serum. The plates are incubated at 37°C, 5% $CO_2$ in a humidified environment for 24 hrs until the desired confluency of 20-30% is achieved. Heat inactivated canine serum samples, positive control and negative control sera are serially diluted 2-fold to appropriate levels in Eagles minimal essential medium. Each test well of the diluted serum is combined with an equal volume of virus suspension containing 100-316 tissue culture infectious dose 50% ($TCID_{50}$) of canine parainfluenza virus. Typically, one can use a non-cytopathic virus. The serum/virus mixture is incubated for an hour to allow neutralization of virus by specific antibody. Challenge virus is titrated to verify the amount of virus used in the assay (back-titration). Following incubation, the fluid contents of the plates are removed and the plates are inoculated with the serum/virus mixture, four replicates per serum dilution. Plates are incubated for $6\pm1$ days at 37°C, 5% CO2 in a humidified environment. The plates are then fixed with 80% acetone, rinsed and stained with direct fluorescein isothiocyanate labeled anti-CPI polyclonal antibody. Following the staining procedure, the plates are read for the presence or absence of viral specific fluorescence. Wells exhibiting no FA are scored as a "+" denoting the presence of neutralizing antibody. Wells containing FA are scored as a "-" denoting the lack of neutralizing antibody. Serum neutralizing titer is calculated by Spearman-Karber. An assay must fulfill the following criteria to be considered valid: 1. The backtiter must be within 100-316 $TCID_{50}$. 2. The positive control serum must be within the established range (typically, 32-82). 3. The negative control serum must have a titer of $\leq2$.

Serological analysis of parvovirus

Norden Laboratories Dog Kidney cells of passage range 126-143 are plated at 16,000 cells per plate well in Eagles minimal essential medium/10% Fetal bovine serum. The plates are incubated at 37°C, 5% $CO_2$ in a humidified environment for 24hrs until the desired confluency of 20-30% is achieved. Heat inactivated canine serum samples, positive control and negative control sera are serially diluted 2-fold to appropriate levels in Eagles minimal essential medium. Each test well of the diluted serum is combined with an equal volume of virus suspension containing 100-316 tissue culture infectious dose 50% ($TCID_{50}$) of canine parvovirus. Typically, one can use a non-cytopathic virus. The serum/virus mixture is incubated for an hour to allow neutralization of virus by specific antibody. Challenge virus is titrated to verify the amount of virus used in the assay (back-titration). Following incubation, the fluid contents of the plates are removed and the plates are inoculated with the serum/virus mixture, four replicates per serum dilution. Plates are incubated for 3-4 days at 37°C, 5% CO2 in a humidified environment. The plates are then fixed with 80% acetone, rinsed and stained with direct fluorescein isothiocyanate labeled anti-CPV polyclonal antibody. Following the staining procedure, the plates are read for the presence or absence of viral specific fluorescence. Wells exhibiting no FA are scored as a "+" denoting the presence of neutralizing antibody. Wells containing FA are scored as a "-" denoting the lack of neutralizing antibody. Serum neutralizing titer is calculated by Spearman-Karber. An assay must fulfill the following criteria to be considered valid: 1. The back-titer must be within 100-316 $TCID_{50}$ 2. The positive control serum must be within the established range (typically, 88-352). 3. The negative control serum must have a titer of $\leq2$.

Serological analysis of canine coronavirus

Norden Laboratories Feline Kidney cells of passage range 85-92 are plated at 50,000 cells per plate well in Eagles minimal essential medium+10% Fetal bovine serum. The plates are incubated at 37°C, 5% $CO_2$ in a humidified environment for $3\pm1$ days until the desired confluency of 100% is achieved. Heat inactivated canine serum samples, positive control and negative control sera are serially diluted 2-fold to appropriate levels in BME+10mM N-(2-hydroxyethyl) piperizine-N'-(2-ethanesulfonic acid)+4$\mu$g/mL polybrene. Each test well of the diluted serum is combined with an equal volume of virus suspension containing 100-316 tissue culture infectious dose 50% ($TCID_{50}$) of canine coronavirus. The serum/virus mixture is mixed and incubated for an hour to allow neutralization of virus by specific antibody. Challenge virus is titrated to verify the amount of virus used in the assay (back-titration). Following incubation, the fluid contents of the plates are removed and the plates are inoculated with serum/virus mixture, four replicates per serum dilution. Plates are incubated for $5\pm1$ days at 37°C, 5% $CO_2$ in a humidified environment. The wells are then read for the presence of virus by cytopathic effect (CPE). Wells exhibiting no CPE are scored as a "+" denoting the presence of neutralizing antibody. Wells containing CPE are scored as a "-" denoting the lack of neutralizing antibody. Serum neutralizing titer is calculated by Spearman-Karber. An assay must fulfill the following criteria to be considered valid: 1. The back-titer must be within 100-316 $TCID_{50}$ 2. The positive control serum must be within the established range (typically, 27-108). 3. The negative control serum must have a titer of $\leq2$.

Serological analysis of *Leptospira canicola*

Living late logarithmic or early stationary phase cultures of *Leptospira canicola* are use for the assay. Heat inactivated canine serum samples, positive control and negative control sera are serially diluted 2-fold to appropriate levels in diluent. An equal volume of culture is added to the diluted serum. The serum/bacterial mixture is mixed and incubated for 2 to 4 hour to allow agglutination of bacteria by specific antibody. The suspension in each well is examined microscopically for agglutination. The endpoint is the highest well in which 50% agglutination is recorded, measured by comparison of the density of unagglutinated leptospires with a control suspension. An assay must fulfill the following criteria to be considered valid: 1. The number of bacteria must used to challenge each well must be between $2 \times 10^8$ and $1 \times 10^9$/mL. 2. The positive control serum must be within the established range. 3. The negative control serum must have a titer of $\leq2$.

Serological analysis of *Leptospira icterohaemorrhagiae*

Living late logarithmic or early stationary phase cultures of *Leptospira icterohaemorrhagiae* are use for the assay. Heat inactivated canine serum samples, positive control and negative control sera are serially diluted 2-fold to appropriate levels in diluent. An equal volume of culture is added to the diluted serum. The serum/bacterial mixture is mixed and incubated for 2 to 4 hour to allow agglutination of bacteria by specific antibody. The suspension in each well is examined microscopically for agglutination. The endpoint is the highest well in which 50% agglutination is recorded, measured by comparison of the density of unagglutinated leptospires with a control suspension. An assay must fulfill the following criteria to be considered valid: 1. The number of bacteria must used to challenge each well must be between $2 \times 10^8$ and $1 \times 10^9$/mL. 2. The positive control serum must be within the established range. 3. The negative control serum must have a titer of $\leq 2$.

Examples

The following examples further illustrate the methods of the present invention. It is to be understood that the present invention is not limited to the specific details of the examples provided below.

EXAMPLE 1: A retrospective study to determine the duration of adequate immune memory of the various viral and bacterial components of Vanguard® 5/L or Vanguard Plus® 5/L vaccines in dogs by measuring antibody titers and antibody response to revaccination.

1. VACCINE

Generic Name: Canine distemper, adenovirus type 2, parvovirus, parainfluenza vaccine, *modified* live virus vaccine (MLV) *Leptospira canicola-icterohaemorrhagiae* bacterin(inactivated whole cultures)

Trade Name: Vanguard® Plus 5/L (APHIS Product Code 4639.22). US Vet. License No.: 189

Formulation: Commercial vaccine

2. STUDY ANIMALS:

| | | | | |
|---|---|---|---|---|
| Species | Canine | Type: | Mixed and purebred dogs | |
| Breed/Strain: | Any | Sex: | M & F (intact or neutered) | |
| Description: | Adults | | | |
| Origin: | Privately owned veterinary patients | | | |
| Initial Age: | Min: 2 | Max: None | Units: Years | |
| Weights: | Min: None | Max: None | Units: Pounds | |

3. MANAGEMENT:

a. Site Specifications

This study is a controlled clinical study conducted at multiple sites over diverse geographic regions in the United States and Canada. One protocol common to all sites isused. Approximately 40-60 veterinary practices will participate in the study. Each individual study site is expected to enroll a minimum of 5 dogs.

The sites are smallanimal veterinary practices whose clientele, record keeping and compliance will provide a large proportion of enrolled patients. The practices should have at least one veterinarian (the Study Director or Examining Veterinarian designated by the Study Director) who is experienced in general veterinary practice and medicine. The practices will have a history of using Vanguard® 5/L or Vanguard Plus® 5/L over the last five years. The owners of the dogs enrolled in the study shall be willing to participate in the study until completion of all observations and collection of all samples.

b. Pretreatment

Medical management will follow normal accepted clinical practice for each study animal. Treatments listed under "Exclusion Criteria" will not be administered and the use of other concomitant medications are recorded.

c. Identification Method

Individual animal identification as provided by owners and unique case report numbers.

d. Housing

Dogs are kept in normal domestic environments and can be housed either in the client's house or in separate animal accommodation. Dogs can be housed with or without other animals.

e. Feeding and Watering Methods

Normal practice.

4. STUDY DESIGN:

| No. | Treatment Description | Time Since Last Vaccination | Route | Animals/ Treatment |
|---|---|---|---|---|
| T1. | Vanguard Ptus® 5/L | ≥12 months | Subcutaneous | Approx. 500-1000 |

5. PROCEDURES:

Dogs are selected for the study from animals presented to each site. The medical records of each site are reviewed to identify adult dogs that have received a priming vaccination series, but have not been vaccinated for 12 months or longer. The most recently administered vaccine is Vanguard® 5/L or Vanguard® Plus 5/L. An attempt is made to identify a range of animals that have not been vaccinated in 12 to 48 months (or longer), such that immune status can be assessed over an extended period since last vaccination. Duration of adequate immune memory to the individual Vanguard® vaccine components are determined in the selected dogs based on serum antibody titers and/or an anamnestic response to revaccination with Vanguard® Plus 5/L.

General information for all identified candidate cases are recorded and eligibility assessed. Only animals that satisfy all of the inclusion criteria and for which none of the exclusion criteria apply are selected for enrollment in the study.

Healthy dogs, 2 years of age or older, which have not been vaccinated in the past 12 months, or longer, are eligible for enrollment. Owners of these animals are contacted by telephone and asked to participate in the study. Reasons for non-participation in the study are recorded.

a. Inclusion Criteria

1. Any breed, sex or weight of dog.
2. Healthy dogs, 2 years of age or older.
3. Dog must not have received a mono- or polyvalent vaccine containing canine distemper adenovirus type 2, parvovirus, parainfluenza vaccine, modified live virus vaccine; *Leptospira canicola-icterohaemorrhagiae* bacterin within the past 12 months, or longer. (Dogs may have received rabies, *Bordetella, Borrelia,* or *Giardia* vaccinations).
4. Dog must have a documented history of vaccination with at least two doses of a polyvalent vaccine (containing canine distemper, adenovirus type 2, parvovirus, parainfluenza vaccine, modified live virus vaccine; *Leptospira canicola-icterohaemorrhagiae* bacterin, from any manufacturer) as a priming dose (puppy or initial vaccination series), with the last dose given when the animal was 12 weeks of age or older.
5. Dog must have received at least one additional polyvalent vaccine 9-14 months after the initial priming series.
6. The most recent vaccine administered must be Vanguard® 5/L or Vanguard Plus® 5/L.
7. The owner/authorized agent must agree to return the study animal to the veterinary practice for evaluation at the required times.
8. The owner/authorized agent must give written informed consent for the animal to participate in the study.

9. The owner must complete the animal lifestyle and disease risk assessment questionnaire.

b. Exclusion Criteria

1. Dogs with a history of intolerance/allergy to vaccinations.
2. Pregnant animals.
3. Dogs with severe systemic disease, whether or not it is a constant threat to life (renal failure, hepatic failure, terminal neoplasia).
4. Dogs treated with any immunosuppressive drugs (corticosteroids including prednisone, and dexamethasone, or other immunosuppressives such as cyclophosphamide, azathioprine, and gold sodium thiomalate, *etc.*) within the past 10 days.
5. Dogs treated with repository anti-inflammatory drugs (*e.g.*, methyl-prednisolone acetate) within the last 30 days.

c. Pre-Vaccination Examination. Blood Collection and Processing

On the enrollment date all dogs will receive a physical examination to determine the dog's general health. The owner will complete a questionnaire to document the lifestyle and risk of disease exposure of their dog.

On the enrollment date (once the dog is confirmed to meet inclusion criteria), approximately ten (10) mL of blood is obtained from the jugular vein (or other peripheral vein) prior to vaccination. Blood is collected into a 12 ml serum separator tube and allowed to clot. Blood is centrifuged, serum removed and aliquoted into two plastic shipping tubes (Replicate A and B). Serum samples are stored frozen prior to shipment. The plastic tubes are labeled with the animal identification number, replicate ID and the date of collection. Laboratory submission forms (supplied by laboratory) and chain-of-custody forms are completed for each sample submission. Frozen samples (Replicate A) are shipped, on ice, weekly to a predetermined laboratory. Upon receipt confirmation of the primary sample, retainer samples (Replicate B) will also be shipped on ice to the participating laboratory. The laboratory are masked to the animal's vaccination history. Dates of blood sample collection and vaccination are recorded.

One laboratory is identified to analyze all serologic samples. The laboratory is required to standardize and validate all assays in order to meet regulatory requirements.

d. Treatment Administration

On Day 0, after collection of pre-vaccination serum samples, dogs are administered one dose (1 mL) of Vanguard Plus® 5/L as per label directions.

e. Post-Vaccination Blood Collection and Processing

Three to 7 days (Study Day 4 - 6) following vaccination, the dog is re-examined and a second blood sample obtained and processed (as described above for pre-vaccination samples) and recorded. Once the post-vaccination sample is collected, the dog is considered off the study.

f. Serological Titers

The following antibody titers are determined for each serum sample: canine distemper virus serum neutralizing ("SN") titer; canine parvovirus SN titer; canine adenovirus type 1 SN titer; canine adenovirus type 2 SN titer; canine parainfluenza virus SN titer; *Leptospira interrogans* ser. *icterohaemorrhagiae* microscopic agglutination ("MA") titer; *Leptospira interrogans* ser. *canicola* MA titer.

The priority for sample analysis is as follows:

1. Canine distemper virus SN titer;
2. Canine parvovirus SN titer;
3. Canine adenovirus type 1 SN titer;
4. Canine adenovirus type 2 SN titer;
5. *Leptospira interrogans* ser. *icterohaemorrhagiae* MA titer;
6. *Leptospira interrogans,* ser. *canicola* MA titer;
7. Canine parainfluenza virus SN titer.

g. Blood Collection and Processing

1. Collect approximately 10 mL of blood into a serum separator (red top) vacutainer tube. Blood can be collected either by syringe, or directly into the vacutainer tube.
2. Allow blood to clot a minimum of 20 minutes, but no more than 40 minutes.
3. Centrifuge clotted blood for a minimum of 5 minutes at appropriate speed.
4. Remove red rubber stopper from separator tube, and carefully pour or pipette serum in two approximately equal aliquots into two plastic shipping tubes. Close the plastic shipping tubes with the cap provided, and apply labels with animal name, animal identification number, and date of collection. Freeze samples at approximately -20°C. If a pipette is used to transfer serum, a new pipette should be used for each sample.
5. Blood should be processed and frozen within two hours of collection.

h. Concurrent Disease/Medication

If medication, medical or surgical procedures are deemed necessary, then standard veterinary practice procedures are followed. No concurrent medications (other than defined permissible concurrent medications), medical or surgical procedures are permitted without prior consent of the Study Director, with the exception of extreme emergencies when even short delays would be life-threatening or cause undue suffering. Animals can be removed from the study, if warranted.

Dogs can be treated with the following medications during the course of the study with the use documented to include drug, dosage, duration and reason for use:

1. Daily or monthly heartworm preventative (*e.g.*, selamectin, diethylcarbamazine, ivermectin, milbemycin)
2. Monthly oral flea preventative (*e.g.*, lufenuron)
3. Topical flea treatment (*e.g.*, selamectin, imidacloprid, organophosphate or pyrethroid based sprays, dips, or powders)
4. Prescription diets
5. Oral antihelminthics (*e.g.*, pyrantel, albendazole, fenbendazole)
6. Medications to control underlying medical disorders (*e.g.*, antimicrobials, thyroid supplementation) can be used if use is documented to identify drug, dosage, duration, dates and reason for use.

6. DATA MANAGEMENT AND STATISTICAL ANALYSIS:

Animals are categorized into groups based on the period of time between their last vaccination and their entry into the study as follows:

| | |
|---|---|
| 12-18 | months |
| 19-24 | months |
| 25-30 | months, etc. |

An animal is put into one of 4 risk categories based on the Lifestyle and Exposure Risk Questionnaire. The categories are low exposure to other dogs and low exposure to other disease vectors (low-low), low exposure to other dogs and high exposure to other disease vectors (low-high), high exposure to other dogs and low exposure to other disease vectors (high-low), and high exposure to other dogs and high exposure to other disease vectors (high-high).

In this application the term "high risk animal", whether applied to dogs, cats or another animal, applies to any animal meeting the criteria for inclusion in either the high-low or high-high categories, while the term "low risk animal" applies to any animal meeting the criteria for inclusion in either the low-low or low-high categories".

a. Lifestyle and Exposure Risk Questionnaire

1. Which of the following best describes the area where you and your dog live? (check one)
□ city
□ suburb

| |
|---|
| □ town, village, or hamlet<br>□ farm or ranch |
| 2. In years, how long have you lived in your present home? |
| 3. In total, how many dogs are in your household? (If there is only one dog in your household, go to question 4.) |
| 3a. How many of these dogs are one year of age or older? |
| 3b. How many of these dogs are less than one year of age? |
| 3c. Have any new dogs been acquired during the last year? |
| 4. During the past year, did you take your dog outdoors to exercise, urinate or defecate? If the answer is no, go to question 5.<br>□ yes<br>□ no |
| When you took/let your dog outdoors, did you:<br>4a. Confine your dog to a kennel, fenced area, or yard?<br>□ yes<br>□ no |
| 4b. Tether your dog to a run, tree, post, or stake?<br>□ yes<br>□ no |
| 4c. Walk your dog on a leash?<br>o yes<br>o no |
| 4d. Allow your dog to roam free with supervision (*i.e.*, your dog is visible at all times)?<br>□ yes<br>□ no |
| 4e. Allow your dog to roam free without supervision?<br>□ yes<br>□ no |
| 4f. Walk or exercise your dog in a park, field, or other area where other dogs are walked or exercised?<br>□ yes<br>□ no |
| During the past year, when your dog was outdoors, did it:<br>4g. Walk or exercise or play with other dogs in your household?<br>□ yes<br>□ no |
| 4h. Walk or exercise or play with other dogs not in your household?<br>□ yes<br>□ no |
| 4i. Ever fight, kill, or share food or water bowls with a wild animal?<br>□ yes<br>□ no |
| 4j. Ever drink from puddles of water, ponds, streams, rivers, or lakes??<br>□ yes<br>□ no |
| 4k. Ever swim in ponds, streams, rivers, or lakes?<br>□ yes<br>□ no |
| 41. Ever eat garbage, refuse, animal carcasses, or trash? |

| |
|---|
| □ yes<br>□ no |
| 4m. Ever eat another dog's feces?<br>□ yes<br>□ no |
| 5. Did you ever take dog to a dog show or field trials?<br>□ yes<br>□ no |
| 6. Did you ever take your dog to obedience classes ?<br>□ yes<br>□ no |
| 7. Did you ever take your dog to a boarding kennel?<br>□ yes |
| □ no |
| 8. Did you ever have your dog groomed by a professional groomer?<br>□ yes<br>□ no |

An animal is put in the low-low risk category if the answer to question 3 is "1", the answer to question 4 is "No" and the answers to questions 5,6, 7 and 8 are "No" or if the answer to question 3 is "1", the answer to question 4 is "Yes", the answer to at least one of questions 4A, 4B or 4C is "Yes" and the answers to questions 4D, 4E, 4F, 4G, 4H, 41, 4J, 4K, 4L, 4M, 5,6,7 and 8 are "No".

An animal is classified as low-high if the answer to question 3 is "1", the answer to question 4 is "Yes", the answer to at least one of questions 4A, 4B or 4C is "Yes", the answers to questions 4D and 4E are "No", the answer to question 4F is "Yes", the answers to questions 4G and 4H are "No", the answer to at least one of questions 41, 4J, 4K, 4L and 4M is "Yes" and the answers to questions 5,6,7 and 8 are "No".

An animal is put in the high-low category if the answer to question 3 is "1", then answer to question 4 is "No", the answer to at least one of questions 5,6,7 or 8 is "Yes", or if the answer to question 3 is "> 1" the answer to question 4 is "Yes", the answer to at least one of questions 4A, 4B or 4C is "Yes", the answers to questions 4D, 4E and 4F is "No", the answer to at least one of questions 4G, 4H, 5, 6,7 or 8 is "Yes" and the answers to questions 41, 4J, 4K, 4L and 4M are "No".

The animal is classified as high-high if the answer to question 3 is ">1", the answer to question 4 is "Yes", the answer to at least one of questions 4D and 4E is "Yes", the answer to question 4F is "Yes", the answer to at least one of questions 4G, 4H, 5, 6, 7 and 8 is "Yes", and the answer to at least one of questions 41, 4J, 4K, 4L and 4M is "Yes".

7. Determining Whether the Animal is Immune

In regions where the clinic records indicate active disease prevalence (or local diagnostic lab data confirms active disease cases), animals that are classified as other than low risk and which have no history of the disease of interest are also to be considered immune (this being considered evidence of immunity in that they remained healthy even when exposed to disease in the environment.) In regions of low (or zero) disease prevalence animals of any risk level will be assigned immune/not immune status based upon the residual post vaccination antibody titers and anamnestic responses.

An animal is considered to be immune to a specific disease if the animal has antibody levels indicating an ongoing immune response for that disease at the time of entry into the study and/or an anamnestic response is induced for that disease by 3-7 days post-vaccination and the animal has no previous history of having that disease. Antibody titers that indicate an ongoing immune response are defined as follows:

a) Canine distemper virus SN titer $\geq$ 1:16
b) Canine parvovirus SN titer $\geq$ 1:16
c) Canine adenovirus, type 1 SN titer $\geq$ 1:16
d) Canine adenovirus, type 2 SN titer $\geq$ 1:16
e) Canine parainfiuenza Virus SN titer $\geq$ 1:4

f) *Leptospira interrogans*, ser. *icterohaemorrhagiae* MA titer ≥ 1:8

g) *Leptospira interrogans* ser. *canicola* MA titer ≥ 1:8

An anamnestic response is defined as an increase in antibody titers of two or more doubling dilutions (four-fold increase) between the blood sample obtained before revaccination and the blood sample obtained 3 - 7 days after revaccination.

The relationship between pre-vaccination time and immune/not immune status is used to assess duration of adequate immune memory for each disease using the logistic regression model discussed above. Only animals with both the first and second blood samples are included in the analysis. Estimates from the logistic regression model are used to determine the duration of adequate immune memory for each antigen as defined as the predicted prevaccination time-point where the percentage of immune animals is ≥80%.

8. Adverse Experience Reporting:

All dogs entered into the study and administered vaccine are observed by the veterinarian and pet owner for the occurrence of any abnormal clinical signs during the following 10 days.

The Study Director will document any telephone reports of unexpected or abnormal clinical signs made by owner/agents and will, based on professional judgement, determine if the abnormal clinical signs observed warrant an unscheduled examination of the animal.

In the unlikely event that an animal should die for any reason during the course of the study, a necropsy should be conducted by a veterinarian or a veterinary pathologist to determine the cause of death.

EXAMPLE 2: A prospective study to determine the duration of adequate immune memory of the various viral and bacterial components of Felocell® CVR (generic name: feline panleukopenia-herpesvirus-calicivirus, modified live virus vaccine) in cats by measuring antibody titers, disease incidence, and assessing risk of exposure to disease over time.

1. Study Animals:

| Species | Feline | Type: | Any |
| Breed/Strain: | Any | Sex: | Male or Female (intact or neutered) |
| Description: | Adults | | |

| Origin: | Privately owned veterinary patients | | | | |
| Initial Age: | Min: | 2 | Max: | 3 | Units: Years |
| Weights: | Min: | None | Max: | None | Units: Pounds |

2. MANAGEMENT:

a. Site Specifications

This study is a controlled clinical trial conducted at multiple sites over diverse geographic regions in the United States and Canada. One protocol common to all sites is used. Approximately 80-100 veterinary practices will participate in the trial. Each individual study site is expected to enroll a minimum of 25 to 30 cats.

The sites are small-animal veterinary practices whose clientele, record keeping and compliance will provide a large proportion of enrolled study animals that satisfactorily complete the study. The owners of the cats shall be willing to participate in the study until completion of all observations and collection of all samples. The practices should have at least one veterinarian (the Study Director or Examining Veterinarian designated by the Study Director) who is experienced in general veterinary practice and medicine.

The practices will have a history of using Felocell® CVR over the last three years.

b. Pretreatment

Medical management will follow normal accepted clinical practice for each study animal. The use of concomitant medications is recorded.

c. <u>Identification Method</u>

Individual animal identification is provided by owners and unique case report numbers.

d. <u>Housing</u>

Cats are kept in normal domestic environments and can be housed either in the client's house or in separate animal accommodation. Cats can be housed with or without other animals.

e. <u>Feeding and Watering Methods</u>

Normal practice.

3. STUDY GROUPS

Cats are selected for the study from animals presented to each veterinary practice such that different risk groups are represented. The medical records of each site are reviewed to identify adult cats that satisfy all of the inclusion criteria and for which none of the exclusion criteria apply.

Animals are assigned to study groups T01 or T02. Animals selected for study group T01 will serve as positive controls and will receive vaccinations annually based on current label recommendations for Felocell® CVR. Animals selected for study group T02 will not be re-vaccinated, but will have antibody titers and incidence of disease monitored throughout the study. On Day 0, disease history and disease risk analysis are recorded and a complete physical examination performed for each animal. Also on Day 0, cats in T01 are administered one dose of Felocell® CVR subcutaneously as per label directions.

| No. | Time Since Last Vaccination | Route | Vaccination Schedule | Animals |
|---|---|---|---|---|
| T01 | Approx. 12-24 months | Subcutaneous | annually | Approx. 500 |
| T02 | Approx. 12-24 months | N/A | N/A | Approx. 1000 |

a. <u>Inclusion Criteria For Study Group T01</u>

1. Any breed, sex or weight of cat.
2. Healthy cats, 2 - 3 years of age.
3. Cat has been vaccinated with Felocell® CVR within the past 11-13 months. Cats may have received rabies, Feline Leukemia, Feline Infectious Peritonitis or *Bordetella* vaccinations within the past 12-months.
4. Cat must have a history of vaccination with a minimum of two doses of a Felocell® CVR given as a kitten as a priming dose, with the last dose administered when the animal is 12- 16 weeks of age or older.
5. Cat must have received at least one additional dose of Felocell® CVR 9 - 14 months after the initial priming series.
6. The name product types and serial numbers of the Felocell® CVR vaccines used and the dates of vaccination must be recorded in the study animal medical record.
7. The owner/authorized agent must agree to return the study animal to the veterinary practice for evaluation and sample collection at the required times.
8. The owner/authorized agent must give written informed consent for the animal to participate in the study.
9. The owner must complete the disease history and disease risk analysis questionnaire at each scheduled visit or by phone (Appendix IV, Form 7).

b. <u>Inclusion Criteria For Study Group T02</u>

1. Any breed, sex or weight of cat

2. Healthy cats, 2 -3 years of age.

3. Cat has not been vaccinated with Felocell® CVR within the past 12-24 months. Cat must not have received a mono- or polyvalent vaccine containing feline panleukopenia-herpesvirus-calicivirus and *Chiamydia psittaci* within the past 11 - 25 months. Cats may have received rabies, Feline Leukemia, Feline Infectious Peritonitis or *Bordetella* vaccinations within the past 12-months.

4. Cat must have a history of vaccination with a minimum of two doses of a Felocell® CVR given as a kitten as a priming dose, with the last dose administered when the animal is 12 - 16 weeks of age or older.

5. Cat must have received at least one additional dose of Felocell® CVR, 9 - 14 months after the initial priming series.

6. The name product types and serial numbers of the Felocell® CVR vaccine used and the dates of vaccination must be recorded in the study animal medical record.

7. The owner/authorized agent must agree to return the study animal to the veterinary practice for evaluation and sample collection at the required times.

8. The owner/authorized agent must give written informed consent for the animal to participate in the study.

9. The owner must complete the risk analysis questionnaire at each scheduled visit or by phone.

c. Exclusion Criteria for All Cats

1. Cats with a history of intolerance/allergy to vaccinations.

2. Pregnant animals.

3. Cats with severe systemic disease, whether or not it is a constant threat to life (renal failure, hepatic failure, terminal neoplasia).

4. Cats treated with any immunosuppressive drugs (corticosteroids including prednisone, and dexamethasone, or other immunosuppressives such as cyclophosphamide, azathioprine, and gold sodium thiomalate, *etc.)* within the past 10 days.

5. Cats treated with repository anti-inflammatory drugs (*e.g.*, methyl-prednisolone acetate) within the last 30 days.

6. Cats that are FeLV or FIV positive on ELISA test.

4. RISK ANALYSIS QUESTIONAIRE

Animals are placed into one of two risk categories based on the lifestyle and disease according to the following questions answered by the pet owner.

| |
|---|
| 1. Which of the following best describes the area where you and your cat live? (check one)<br>☐ city<br>☐ suburb<br>☐ town, village, or hamlet<br>☐ farm or ranch |
| 2. In years, how long have you lived in your present home? |
| 3. In total, how many cats are in your household? (if there is only one cat in your household, go to question 4.) |
| 3a. How many of these cats are one year of age or older |
| 3b. How many of these cats are less than one year of age? |
| 3c. Have any new cats been acquired during the last year? |
| 4. Which of the following best describes the housing of your cat(s)? (check one)<br>☐ All cats are indoors 100% of the time<br>☐ One or more cats go outside but not every day<br>☐ One or more cats goes outside every day<br>☐ One or more cats are outdoors 100% of the time |
| 5. Which of the following best describes the housing of the cat here today?(check one)<br>☐ All cats are indoors 100% of the time<br>☐ One or more cats go outside but not every day |

| |
|---|
| ☐ One or more cats goes outside every day<br>☐ One or more cats are outdoors 100% of the time |
| 6. Did you board your cat in a boarding kennel any time during the fast year?(cheek one)<br>☐ yes<br>☐ no |
| 7. Did your cat ever escape from the house and was lost or missing for more than 24 hours?(check one)<br>☐ yes<br>☐ no |
| 8. Did you take your cat to cat shows?(check one)<br>☐ yes<br>☐ no |

An animal is placed in the low-risk category if the answer to question 3 is "1", the answer to question 5 is "100% indoors" and the answers to questions 6, 7, and 8 are "No" or if the answer to question 3 is "2" or "3", the answer to question 3c is "No", the answers to questions 4 and 5 are "100% indoors" and the answers to questions 6, 7, and 8 are "No". Otherwise the animal is categorized as being in the high-risk category. The risk category is determined for every time a questionnaire is completed. For purposes of statistical analysis, once an animal is categorized as high in either the exposure to other cats or exposure to other disease vectors it cannot be reclassified as low in that category.

5. PROCEDURES

a. Examinations, Serum Collection and Vaccination for the T01 Study Group

Following initial examination on the enrollment date, owners of cats in study group T01 are interviewed in person or by phone at 6 month (26±2 weeks) intervals for the duration of the study to determine disease history and conduct disease risk analysis. Disease history and disease risk analysis will occur in person at the first 6-month exam and again at the 1-year exam because cats are required to visit the participating clinic for blood sample collection to determine serum antibody titers. Blood samples will not be collected beyond year one from cats in T01. The cats are administered one dose of Felocell® Plus® 5/L subcutaneously as per label directions at each 12-month ($52 \pm 2$ weeks) interval for the duration of the study.

b. Examinations and Serum Collection for the T02 Study Group

For cats in the T02 study group, on the enrollment date, disease history and disease risk analysis are recorded and a physical examination is performed for each animal. Also on the enrollment date, (once the cat is confirmed to meet inclusion criteria), approximately, five (5) mL of whole blood will be obtained from the jugular (or other peripheral) vein.
Cats in the T02 study group are then re-examined at 6-month ($26 \pm 2$ weeks) intervals following the initial examination for the duration of the study. At each 6-month visit, disease history and disease risk analysis will be recorded, a physical examination performed, and a blood sample collected for the determination of serum antibody titers. When serum antibody titers drop below defined levels for any one antigen, the animal will receive a monovalent vaccine containing that antigen only.

c. Serum Sample Processing

On the enrollment date, approximately, five (5) mL of blood is obtained from the jugular (or other peripheral vein) prior to vaccination. One half (0.5) mL of whole blood is used to test for the presence of FELV antigen and FIV antibody using a standard combination ELISA test kit and the results recorded on the animal physical examination form. The remainder of the blood is collected into a 7 mL serum separator tube (red top) and allowed to clot. If the results of the FELV and FIV ELISA are negative and the cat is confirmed to meet the inclusion criteria, then blood samples are centrifuged, then the serum is removed and aliquoted into two plastic shipping tubes (Replicates A and B). All serum samples are stored frozen prior to shipment. The plastic tubes are labeled with the animal identification number, replicate ID and the date of collection. Laboratory submission forms (supplied by laboratory) and chain-of-custody forms are completed for each sample submission.
Frozen samples (Replicate A) are shipped, on dry ice, weekly, to a predetermined laboratory. Upon receipt confirmation of the primary sample, retainer samples (Replicate B) will also be shipped on ice to the participating laboratory. The laboratory is masked to the animal's vaccination history. Dates of blood sample collection and vaccination are recorded.

One laboratory is identified to analyze all serologic samples. The laboratory will standardize and validate all assays to meet regulatory requirements prior to initiation of sample analysis.

d. Blood Collection And Processing

1. Collect approximately 5 mL of blood into a serum separator (red top) vacutainer tube. Blood can be collected either by syringe, or directly into the vacutainer tube.
2. Allow blood to clot a minimum of 20 minutes, but no more than 40 minutes.
3. Centrifuge clotted blood for a minimum of 5 minutes at appropriate speed.
4. Remove red rubber stopper from separator tube, and carefully pour serum into plastic shipping tube. Close the plastic shipping tube with the cap provided, and apply label with animal name, animal identification number, and date of collection. Freeze sample. If a pipette is used to transfer serum, a new pipette should be used for each sample.
5. Blood should be processed and frozen within two hours of collection.

e. Serological Titers

The following antibody titers are determined for each serum sample: feline panleukopenia virus serum neutralizing (SN) titer; feline herpesvirus SN titer; feline calicivirus SN titer; *Chlamydia psittaci* microscopic agglutination (MA) titer. The laboratory will retain all remaining sera until notice from the Study Monitor is given.

The priority for sample analysis is as follows:

1. feline panleukopenia virus SN titer;
2. feline herpesvirus SN titer;
3. feline calicivirus SN titer;
4. *Chlamydia psittaci* MA titer

f. Revaccination of T02 Cats

Laboratory results of serum titers for each are available to the veterinarian 7-10 working days following vaccination. The serum antibody levels are used to determine if additional vaccines need to be administered. When the serum antibody titer for a viral or bacterial antigen decreases below the minimum level that indicates an immune response, the owner is contacted by phone and advised to have the cat revaccinated. The owner will return the cat to be revaccinated with a monovalent vaccine containing that particular antigen. Once revaccinated, the cat will remain in the study, subsequent serum antibody titers measured for the revaccinated antigen will not be included in the analysis.

g. Concurrent Disease/Medication

Cats can be treated with the following medications during the course of the study with the use documented to include drug, dosage, duration and reason for use:

1. Monthly heartworm preventative (*e.g.*, selamectin, ivermectin)
2. Monthly oral flea preventative (*e.g.*, lufenuron)
3. Topical flea treatment (*e.g.*, selamectin, imidacloprid or pyrethroid based sprays, dips, or powders)
4. Prescription diets
5. Oral antihelminthics (*e.g.*, pyrantel, albendazole, fenbendazole)
6. Medications to control underlying medical disorders (*e.g.*, antimicrobials, insulin, methimazole) can be used if use is documented to identify drug, dosage, duration, dates and reason for use.

h. Termination of Data Collection

Animals in study groups T01 and T02 are followed for approximately 4 years after entry into the study. Data collection is terminated for a particular antigen when (80%) of the T02 study population no longer maintains antibody titers suggestive of an active immune response. Early termination of the study will occur when (80%) of the T02 study population no longer maintains antibody titers indicating an active immune response against feline panleukopema virus.

6. ADVERSE EXPERIENCE REPORTING:

All cats entered into the study and administered vaccine are observed by the veterinarian and pet owner for the occurrence of any abnormal clinical signs during the following 10 days.

The Study Director will document any reports of unexpected or abnormal clinical signs made by owner/agents and will, based on professional judgement, determine if the abnormal clinical signs observed warrant an unscheduled examination of the animal.

In the event that an animal should die for any reason during the course of the study, a necropsy should be conducted by a veterinarian or a veterinary pathologist to determine the cause of death, if possible. Where appropriate, at the discretion of the veterinary pathologist, samples are collected for histopathological and/or microbiological examination and a thorough report of macroscopic and microscopic findings are completed and reported.

7. DATA MANAGEMENT AND STATISTICAL ANALYSIS

In regions where the clinic records indicate active disease prevalence (or local diagnostic lab data confirms active disease cases) animals that are classified as other than low risk and which have no history of the specific disease of interest are also to be considered immune T(his being considered evidence of immunity in that they remained healthy even when exposed to the specific disease in the environment.) In regions of low (or zero) disease prevalence, animals of any risk level will be assigned immune/not immune status based upon the antibody titers and responses.

An animal is considered to be immune to a specific disease if the animal has detectable antibody levels for that disease at the time of blood sampling and no previous history of having that disease. Otherwise, the animal is categorized as not immune. Preferably, the antibody titers that indicate an ongoing immune response are as follows:

a) feline panleukopenia virus SN titer $\geq$ 1:16;
b) feline herpesvirus SN titer $\geq$ 1 :2;
c) feline galicivirus SN titer $\geq$ 1:4;
d) *Chiamydiapsittaci* MA titer $\geq$ 1:8.

If an animal remains immune throughout the study or is immune while it remains in the study, it is recorded as censored and the time until no longer immune recorded as its last available time point. The duration of adequate immune memory is estimated by a duration of adequate Immune memory prediction equation as described above.

### SCHEDULE OF OPERATIONS

#### T01 Vaccinated Control Cats

| WEEK OF STUDY | EVENT/ACTIVITY |
| --- | --- |
| 0 | Physical exam, blood sample, vaccinate, disease history, risk evaluation |
| 26 $\pm$ 2 | Physical exam, blood sample, disease history, risk evaluation |
| 52 $\pm$ 2 | Physical exam, blood sample, vaccinate, disease history, risk evaluation |
| 78 $\pm$ 2 | Physical exam, disease history, risk evaluation |
| 104 $\pm$ 2 | Physical exam, vaccinate, disease history, risk evaluation |
| 130 $\pm$ 2 | Physical exam, disease history, risk evaluation |
| 156 $\pm$ 2 | Physical exam, vaccinate, disease history, risk evaluation |
| 182 $\pm$ 2 | Physical exam, disease history, risk evaluation |
| 208 $\pm$ 2 | Physical exam, vaccinate, disease history, risk evaluation |
| 234 $\pm$ 2 | Physical exam, disease history, risk evaluation |

#### T02 Non-vaccinated Study Cats .

| WEEK OF STUDY | EVENT/ACTIVITY |
| --- | --- |
| 0 | Physical exam, blood sample, disease history, risk evaluation |
| 26 $\pm$ 2 | Physical exam, blood sample, disease history, risk evaluation |

(continued)

| T02 Non-vaccinated Study Cats . | |
| --- | --- |
| WEEK OF STUDY | EVENT/ACTIVITY |
| 52 $\pm$ 2 | Physical exam, blood sample, disease history, risk evaluation |
| 78 $\pm$ 2 | Physical exam, blood sample, disease history, risk evaluation |
| 104 $\pm$ 2 | Physical exam, blood sample, disease history, risk evaluation |
| 130 $\pm$ 2 | Physical exam, blood sample, disease history, risk evaluation |
| 156 $\pm$ 2 | Physical exam, blood sample, disease history, risk evaluation |
| 182 $\pm$ 2 | Physical exam, blood sample, disease history, risk evaluation |
| 208 $\pm$ 2 | Physical exam, blood sample, disease history, risk evaluation. |
| 234 $\pm$ 2 | Physical exam, blood sample, disease history, risk evaluation |

**Claims**

1. A method of determining a duration of adequate immune memory induced by a vaccine for a disease, in an animal where the animal is a dog or a cat, the method comprising:

   (a) selecting a plurality of study animals from one or more clinics,

       i) where each animal has been vaccinated with the vaccine,
       ii) where a time since a last vaccination date is at least about one year
       iii) where the animal has been living in a field environment for at least about one year after the last vaccination date and
       iv) where each animal has a vaccine and health record;
       v) where each animal has been evaluated for risk of exposure to said disease;

   (b) assign said animals a risk category according to the following:

       (i) animals are considered high risk or low risk based on exposure to other animals or disease vectors

           (1) low risk animals are kept indoors and or are NOT exposed to other animals and disease vectors,
           (2) high risk animals are animals not kept indoors and ARE exposed to other animals, or disease vectors,

   (c) assigning each animal an indicator of adequate immune memory, where that indicator is either as a non-immune animal, aka first indicator animal, or an immune animal, aka second indicator animal comprising:

       (i) evaluate said animals for clinical signs of said disease using observations and said health records since last vaccination date,
       (ii) identify evaluated animals having signs of said disease, these are non-immune animals, aka first indicator animals,
       (iii) identify said high risk animals living in areas of disease prevalence and not having signs of said disease determined by clinic records and/or lab confirmation of disease cases in said living area, these are immune animals, aka second indicator animals,
       (iv) evaluate a blood serum sample from each animal that has not shown clinical signs of the disease and that is not a high risk animal living in an area of disease prevalence and not having signs of said disease since the last vaccination date,
       (v) identify those animals having either a cellular immune response and or an antibody titer of at least 2, these are immune animals, aka second indicator animals,
       (vi) identify those animals having no cellular immune response and or an antibody titer less than 2,
       (vii) administer a booster dose of the vaccine to these animals,
       (viii) evaluate a blood serum sample from each animal that has received the booster dose, 3 cays to 28 days following the booster dose,
       (ix) identify those animals having an adequate anamnestic response of at least a 4-fold increase in serum antibody titer, or a statistically significant increase in cellular immune response, these are immune animals, aka second Indicator animals.

(x) identify those animals not having an adequate anamnestic response of a 4-fold increase In serum antibody titer, or a statistically significant Increase in cellular immune response, these are non-immune animals, aka first indicator animals, and

(d) determining the duration of adequate immune memory for said animals, by determining this

from a duration of adequate immune memory estimation equation, said duration of adequate Immune memory estimation equation derived by a logistic regression analysis of the first and the second indicators and the vaccine administration record.

2. A method of according to the method of claim 1 where the final step of claim 1, step d, comprises:

(a) determining an embodiment date for each animal, the enrollment date being when evaluation of the animal to detect the marker of immunity was begun;

(b) assigning a start of study date as the enrollment date for a first animal of the plurality of animals enrolled in the study;

(c) assigning a variable Xj for each animal as a number of days between the start of study date and the enrollment date for the animal;

(d) assigning a variable XI for each animal as a number of days between the last vaccination date for the animal and the enrollment date for the animal.

(e) determining the duration of adequate immune memory from a duration of adequate immune memory estimation equation, said duration of adequate immune memory estimation equation determined by a logistic regression analysis of the first and second indicators and the variables Xj and XI.

3. A method of according to the method of claim 2 where the final determining step of step (e) comprises a memory estimation equation in a form $\text{logit}(E) = \beta 0 + \beta 1 \, XDI + \beta 2 \, XCV + Ck$ where:

E is a desired level of efficacy;
XDI is the duration of adequate Immune memory;
XCV is a mean of XI;
Ck is a constant representing a random effect to account for variation between the clinics and is derived by logistic regression; and
$\beta 0, \beta 1, \beta 2$ are constants derived by logistic regression.

4. The method of claim 3, wherein a model for the logistic regression to derive the values of Ck, $\beta 0$, $\beta 1$, and $\beta 2$ is in a form $\text{logit}(p) = \beta () + \beta 1Xi + \beta 2Xj + Ck$; where:

$\text{logit}(p)$ Is a vector representing the immune statuses for the animals;
Ck is the clinic from which each animal was selected.

5. The method of claim 1, wherein;

(a) the animal is a cat, and
(b) cats are considered high risk or low risk based on questionnaires on exposure to other cats or disease vectors.

6. The method of claim 5, wherein

(a) high risk animals in these areas are considered immune if no clinical signs for disease are observed.
(b) all other animals are considered immune if:

(i) Ab titers determined through serum neutralization are:

$\geq 1:16$ for feline panleukopenia virus,
$\geq 1:2$ for feline herpesvirus,
$\geq 1:4$ for feline calicivirus,

or

(ii) Ab titers determined through microscopic agglutination are:

1:8 for fɛ fine Chtamydia psittaci virus,

(c) when 80% of animals no longer Immune to said disease agent this time point is used as time for end of immunity for logistic regression analysis (duration of adequate immune memory prediction equation) of duration of adequate immune memory.

**7.** The method of claim 1, wherein;

(a) said animal is a dog, and
(b) said dogs are selected from a variety veterinary clinics in distinct geographic regions. The dogs selected will have been vaccinated at least twice with the Vanguard vaccine or a competitor vaccine having the same antigens.
(c) said dogs will have been given a booster dose of the Vanguard vaccine at around 12 (9-14) months
(d) said clinic will have maintained adequate records for each dog, the records documenting date of initial vaccination and any disease instances and associated diagnosis,
(e) upon selecting a plurality of study animals from one or more clinics, enrollment, a questionnaire is filled out by the dog owner describing the lifestyle of the dog (i.e. high to low disease risk),
(f) said dogs are assigned a risk category defined by the frequency of exposure to other dogs or to disease vectors,
(g) upon said enrollment blood is collected, the first blood sample, and antibody titers are determined for all dogs for the vaccine antigens in Vanguard (CDV. CAV-2, CPI, CPV, Leptospira canicola and icterohaemorrhagiae).
(h) after said blood is collected the dog is revaccinated (boosted) and 3-7 days later re-bled to collect a second blood sample,
(i) said second blood sample will be analyzed identically to said first blood sample to quantitate antibody levels,
(j) immune status is determined according to any of the following:

(i) animals classified as anything above low disease exposure risk (i.e. in higher than low risk categories) and that have remained healthy are considered immune, aka second Indicator animals,
(ii) animals that are classed in low or zero disease risk categories and that have retained residual antibody titers (see below), or that have responded to booster within 3-7 days and have no history of disease (see below) will be considered immune, aka second indicator animals,
(iii) Antibody levels that indicate ongoing immunity, aka second indicator animals, are also described as follows;

(1)
(2) CDV SN >/= 1:16
(3) CPV SN >/= 1:16
(4) CAV-1SN >/= 1:16
(5) CAV-2 SN >/= 1:16
(6) CPI SN >/= 1:4
(7) Lepto. ictero >/= 1:8
(8) Lepto canicol >/= 1:8

(k) logistic regression is used to determine duration of immunity by relating time of vaccination to immune or not immune status.

**Patentansprüche**

**1.** Verfahren zur Bestimmung der Dauer eines adäquaten immunologischen Gedächtnisses, induziert durch einen Impfstoff, für eine Krankheit bei einem Tier, wobei das Tier ein Hund oder eine Katze ist, wobei das Verfahren:

(a) die Auswahl einer Vielzahl an Studientieren aus einer oder mehreren Kliniken,

i) wobei jedes Tier mit dem Impfstoff geimpft worden ist,
ii) wobei das letzte Impfdatum mindestens etwa ein Jahr zurückliegt

iii) wobei das Tier für mindestens etwa ein Jahr nach dem letzten Impfdatum in einer Feldumgebung gelebt hat und

iv) wobei jedes Tier eine Impfstoff- und Krankengeschichte hat;

v) wobei jedes Tier hinsichtlich des Risikos einer Exposition zu der Krankheit beurteilt worden ist;

(b) das Zuordnen der Tiere einer Risikokategorie gemäß folgendem:

i) die Tiere werden basierend auf dem Kontakt zu anderen Tieren oder Krankbeitsvektoren als stark gefährdet oder wenig gefährdet betrachtet

(1) wenig gefährdete Tiere werden drinnen gehalten und/oder haben KEINEN Kontakt zu anderen Tieren und Krankheitsvektoren,

(2) stark gefährdete Tiere werden nicht drinnen gehalten und HABEN Kontakt zu anderen Tieren oder Krankheitsvektoren ;

(c) das Zuordnen eines Indikators für ein adäquates immunologisches Gedächtnis jedem Tier, wobei dieser Indikator entweder wie ein nicht-immunes Tier, auch Tier mit erstem Indikator genannt, oder ein immunes Tier, auch Tier mit zweitem Indikator genannt, ist, umfassend:

(i) Bewertung der Tiere hinsichtlich klinischer Anzeichen der Krankheit durch Beobachtung und der Krankheitsgeschichten seit dem letzten Impfdatum,

(ii) Identifizieren der bewerteten Tiere mit Anzeichen der Krankheit, diese sind nicht-immune Tiere, auch Tiere mit erstem Indikator genannt,

(iii) Identifizieren der stark gefährdeten Tiere, die da leben, wo die Krankheit vorherrscht, und ohne Anzeichen der Krankheit, bestimmt durch klinische Aufzeichnungen und/oder Laborbestätigung von Krankheitsfällen in diesem Lebensraum, diese sind immune Tiere, auch Tiere mit zweitem Indikator genannt,

(iv) Bewertung einer Blutserumprobe von jedem Tier, welches keine klinischen Anzeichen der Krankheit gezeigt hat und das kein stark gefährdetes Tier ist, das dort lebt, wo die Krankheit vorherrscht, und keine Anzeichen der Krankheit seit dem letzten Impfdatum zeigt,

(v) Identifizieren der Tiere, die entweder eine zelluläre Immunantwort und/oder einen Antikörpertiter von mindestens 2 haben, diese sind immune Tiere, auch Tiere mit zweitem Indikator genannt,

(vi) Identifizieren der Tiere, die keine zelluläre Immunantwort und/oder einen Anti-körpertiter weniger als 2 haben,

(vii) Verabreichung einer Boosterdosis des Impfstoffes an diese Tiere,

(viii) Bewertung einer Blutserumprobe von jedem Tier, das die Boosterdosis erhalten hat, 3 bis 28 Tage nach der Boosterdosis,

(ix) Identifizieren der Tiere mit einer adäquaten Anamneseanwort mit einer Erhöhung des Serumantikörpertiters um mindestens ein 4-faches, oder einer statistisch signifikanten Erhöhung der zellulären Immunantwort, diese sind immune Tiere, auch Tiere mit zweitem Indikator genannt,

(x) Identifizieren der Tiere ohne adäquate Anamneseanwort mit einer Erhöhung des Serumantikörpertiters um ein 4-faches, oder einer statistisch signifikanten Erhöhung der zellulären Immunantwort, diese sind nicht-immune Tiere, auch Tiere mit erstem Indikator genannt, und

(d) die Bestimmung der Dauer des adäquaten immunologischen Gedächtnisses der Tiere durch Bestimmung dieser aus einer Bestimmungsgleichung für die Dauer des adäquaten immunologischen Gedächtnisses, wobei diese Bestimmungsgleichung für die Dauer des adäquaten immunologischen Gedächtnisses durch logistische Regressionsanalyse des ersten und zweiten Indikators und der Impfstoffverabreichungsgeschichte hergeleitet wird,

umfaßt.

2. Verfahren nach Anspruch 1, wobei der letzte Schritt von Anspruch 1, Schritt d:

(a) die Bestimmung eines Aufnahmedatums für jedes Tier, wobei das Aufnahmedatum das ist, an dem die Bewertung des Tiers hinsichtlich der Detektion des Immunitätsmarkers begann ;

(b) die Festlegung eines Studienbeginndatums als das Aufnahmedatum für ein erstes Tier von mehreren Tieren, die in die Studie aufgenommen wurden ;

(c) die Festlegung einer Variable $X_j$ für jedes Tier als eine Anzahl von Tagen zwischen Studienbeginn und Aufnahmedatum für das Tier;

(d) die Festlegung einer Variable $X_i$ für jedes Tier als eine Anzahl von Tagen zwischen dem letzten Impfdatum für das Tier und dem Aufnahmedatum für das Tier ;

(e) die Bestimmung der Dauer des adäquaten immunologischen Gedächtnisses aus einer Bestimmungsgleichung für die Dauer des adäquaten immunologischen Gedächtnisses, wobei die Bestimmungsgleichung für die Dauer des adäquaten immunologischen Gedächtnisses durch logistische Regressionsanalyse des ersten und zweiten Indikators und der Variablen $X_j$ und $X_i$ hergeleitet wurde, umfaßt.

3. Verfahren nach Anspruch 2, wobei der letzte Bestimmungsschritt von Schritt (e) eine Gedächtnisermittlungsgleichung in folgender Form umfaßt: logit(E) = β0 + β1 XDI + β2 XCV + Ck, wobei:

E das gewünschte Wirksamkeitsniveau ist;

XDI die Dauer des adäquaten immunologischen Gedächtnisses ist;

XCV ein Mittel von Xj ist;

Ck eine Konstante ist, die eine zufällige Wirkung wiedergibt, um Abweichungen zwischen den Kliniken zu berücksichtigen, und durch logistische Regression hergeleitet wurde; und

β0, β1, β2 Konstanten sind, die aus der logistischen Regression hergeleitet wurden.

4. Verfahren nach Anspruch 3, wobei ein Modell für die logistische Regression zur Herleitung der Werte für Ck, β0, β1 und β2 wie folgt lautet logit(p) = β0 + β1Xi + β2Xj + Ck, wobei:

logit(p) ein Vektor ist, der den Immunstatus für die Tiere darstellt, und

Ck die Klinik ist, aus der jedes Tier ausgewählt wurde.

5. Verfahren nach Anspruch 1, worin:

(a) das Tier eine Katze ist und

(b) die Katzen basierend auf Fragebögen zum Kontakt mit anderen Katzen oder Krankheitsvektoren als stark gefährdet oder wenig gefährdet betrachtet werden.

6. Verfahren nach Anspruch 5, worin:

(a) stark gefährdete Tiere in diesen Bereichen als immun betrachtet werden, wenn keine klinischen Anzeichen der Krankheit zu beobachten sind,

(b) alle anderen Tiere als immun betrachtet werden, wenn;

(i) Ab-Titer, bestimmt durch Serumneutralisation:

≥ 1 : 16 für Katzen-Panleukopenie-Virus,

≥ 1 : 2 für Katzen-Herpesvirus,

≥ 1 : 4 für Katzen-Calicivirus,

sind oder

(ii) Ab-Titer, bestimmt durch mikroskopische Agglutination:

≥ 1 : 8 für Katzen-*Chlamydia psittaci*- Virus sind;

(c) 80 % der Tiere nicht länger immun gegen dieses Krankheitsmittel sind, dieser Zeitpunkt als Zeit für das Ende der Immunität für die logistische Regressionsanalyse (Vorhersagegleichung für die Dauer des adäquaten immunologischen Gedächtnisses) zur Dauer des adäquaten immunologischen Gedächtnisses verwendet wird.

7. Verfahren nach Anspruch 1, wobei:

(a) das Tier ein Hund ist und

(b) die Hunde aus einer Vielzahl von Veterinärkliniken in bestimmten geographischen Regionen ausgewählt wurden. Die ausgewählten Hunde mindestens zweimal mit Vanguard-Impfstoff oder einem Konkurrenzimpfstoff mit denselben Antigenen geimpft worden sind;

(c) die Hunde eine Boosterdosis des Vanguard-Impfstoffes bei etwa 12 (9 - 14) Monate erhalten haben;

(d) die Klinik adäquate Berichte für jeden Hund erfaßt hat, wobei die Berichte das Datum der anfänglichen Impfung und jegliche Krankheitsfälle und damit verbundene Diagnosen dokumentiert;

(e) nach der Auswahl der Studientiere aus einer oder mehreren Kliniken, Aufnahme, ein Fragebogen vom Hundebesitzer, der die Lebensumstände des Hundes (d. h. hohes bis geringes Krankheitsrisiko) beschreibt, ausgefüllt wird;

(f) die Hunde einer Risikokategorie, definiert durch die Häufigkeit des Kontaktes zu anderen Hunden oder zu Krankheitsvektoren, zugewiesen werden;

(g) nach der Aufnahme Blut abgenommen wird, die erste Blutprobe, und für alle Hunde Antikörpertiter für die Impfstoffantigene in Vanguard (CDV, CAV-2, CPI, CPV, *Leptospira canicola* und *icterohaemorrhagiae)* bestimmt werden.

(h) nach der Blutabnahme der Hund erneut geimpft (boosted) und 3 - 7 Tage später erneut Blut für eine zweite Blutprobe abgenommen wird;

(i) die zweite Blutprobe identisch zu der ersten Blutprobe analysiert wird, um die Antikörperkonzentrationen zu quantifizieren,

(j) der Immunstatus folgendermaßen bestimmt wird:

(i) Tiere, die oberhalb eines geringen Krankheitsexpositionsrisikos (d. h. in eine Kategorie höher als wenig gefährdet) eingestuft werden und die gesund geblieben sind, werden als immun betrachtet, auch Tiere mit zweitem Indikator genannt,

(ii) Tiere, die als wenig bis gar nicht gefährdet eingestuft werden und die restliche Antikörpertiter beibehielten (siehe unten), oder die innerhalb von 3 - 7 Tagen auf den Booster reagierten und keine Krankheitsgeschichte vorweisen (siehe unten), werden als immun betrachtet, auch Tiere mit zweitem Indikator genannt;

(iii) Antikörperkonzentrationen, die andauernde Immunität anzeigen, auch Tiere mit zweitem Indikator genannt, werden auch folgendermaßen beschrieben:

(1)
(2) CDV SN >/= 1 : 16
(3) CPV SN >/= 1 : 16
(4) CAV-1 SN >/= 1 : 16
(5) CAV-2 SN >/= 1 : 16
(6) CPI SN >/= 1 : 4
(7) Lepto. Ictero >/= 1 : 8
(8) Lepto canicol >/= 1 : 8

(k) die logistische Regression zur Bestimmung der Dauer der Immunität verwendet wird, indem der Zeitpunkt der Impfung mit dem Immun- oder Nicht-Immun-Status in Verbindung gebracht wird.

## Revendications

1. Méthode pour déterminer une durée de mémoire immunitaire adéquate induite par un vaccin, pour une maladie, chez un animal, l'animal étant un chien ou un chat,
méthode comprenant :

(a) la sélection d'une pluralité d'animaux d'étude d'un ou plusieurs milieux cliniques,

i) chaque animal ayant été vacciné avec le vaccin,
ii) le temps depuis la dernière date de vaccination étant d'au moins environ un an,
iii) l'animal ayant vécu dans un environnement naturel pendant au moins environ un an après la dernière date de vaccination et
iv) chaque animal ayant un document de vaccination et de santé ;
v) chaque animal ayant été évalué en ce qui concerne le risque d'exposition à ladite maladie ;

(b) l'attribution auxdits animaux d'une catégorie de risques d'après les critères suivants :

i) les animaux sont considérés comme présentant un haut risque ou un risque bas en fonction de l'exposition à d'autres animaux ou vecteurs de la maladie

(1) les animaux à risque bas sont maintenus dans des locaux ou ne sont PAS exposés à d'autres animaux et des vecteurs de la maladie,

(2) les animaux à haut risque sont des animaux non maintenus dans des locaux et qui SONT exposés à d'autres animaux ou des vecteurs de la maladie,

(c) l'attribution à chaque animal d'un indicateur de mémoire immunitaire adéquate, cet indicateur étant sous forme d'un animal non immun, c'est-à-dire d'un premier animal indicateur, ou bien d'un animal immun, c'est-à-dire d'un second animal indicateur, comprenant :

(i) l'évaluation desdits animaux pour des signes cliniques de ladite maladie en utilisant des observations et lesdits documents de santé depuis la dernière date de vaccination,

(ii) l'identification des animaux évalués présentant des signes de ladite maladie, ces animaux étant des animaux non immuns, c'est-à-dire des premiers animaux indicateurs,

(iii) l'identification desdits animaux à haut risque vivant dans des zones de prévalence de la maladie et ne présentant pas de signes de ladite maladie déterminés par des bilans cliniques et/ou une confirmation en laboratoire de cas de la maladie dans ladite zone de vie, ces animaux étant des animaux immuns, c'est-à-dire des seconds animaux indicateurs,

(iv) l'évaluation d'un échantillon de sérum sanguin provenant de chaque animal qui n'a pas présenté de signes cliniques de la maladie et qui n'est pas un animal à haut risque vivant dans une zone de prévalence de la maladie et ne présentant pas de signes de ladite maladie depuis la dernière date de vaccination,

(v) l'identification des animaux présentant une réponse immunitaire cellulaire et/ou un titre d'anticorps d'au moins 2, ces animaux étant des animaux immuns, c'est-à-dire des seconds animaux indicateurs,

(vi) l'identification des animaux ne présentant aucune réponse immunitaire cellulaire et/ou de titre d'anticorps inférieur à 2,

(vii) l'administration d'une dose de rappel du vaccin à ces animaux,

(viii) l'évaluation d'un échantillon de sérum sanguin provenant de chaque animal qui a reçu la dose de rappel, 3 jours à 28 jours après la dose de rappel,

(xi) l'identification des animaux présentant une réponse anamnestique adéquate d'augmentation d'au moins un facteur 4 du titre d'anticorps sérique, ou une augmentation statistiquement significative de la réponse immunitaire cellulaire, ces animaux étant des animaux immuns, c'est-à-dire de seconds animaux indicateurs,

(x) l'identification des animaux ne présentant pas de réponse anamnestique adéquate d'augmentation d'un facteur 4 du titre d'anticorps sérique, ou d'augmentation statistiquement significative de la réponse immunitaire cellulaire, ces animaux étant des animaux non immuns, c'est-à-dire de premiers animaux indicateurs ; et

(d) la détermination de la durée de la réponse immunitaire adéquate pour les animaux, en déterminant celle-ci d'après une équation d'estimation de durée de mémoire immunitaire adéquate, ladite équation d'estimation de durée de mémoire immunitaire adéquate étant obtenue par une analyse de régression logique des premier et second indicateurs et de l'indication d'administration du vaccin.

2. Méthode suivant la méthode de la revendication 1, dans laquelle l'étape finale de l'étape d de la revendication 1 comprend :

(a) la détermination d'une date de participation pour chaque animal, la date de participation étant la date au moment où l'évaluation de l'animal pour détecter le marqueur ou l'immunité a été commencée ;

(b) l'attribution d'une date de début d'étude comme date de participation pour un premier animal de la pluralité d'animaux amenés à participer à l'étude ;

(c) l'attribution d'une variable Xj pour chaque animal sous forme d'un nombre de jours entre la date de début de l'étude et la date de participation de l'animal ;

(d) l'attribution d'une variable Xi pour chaque animal sous forme d'un nombre de jours entre la dernière date de vaccination de l'animal et la date de participation de l'animal ;

(e) la détermination de la durée de mémoire immunitaire adéquate d'après une équation d'estimation de durée de mémoire immunitaire adéquate, ladite équation d'estimation de durée de mémoire immunitaire adéquate étant déterminée par une analyse de régression logique des premier et second indicateurs et des variables Xj et Xi.

3. Méthode suivant la méthode de la revendication 2, dans laquelle l'étape de détermination finale de l'étape (e) comprend une équation d'estimation de mémoire sous une forme logit (E) = $\beta_0 + \beta_1$ XDI + $\beta_2$ XCV + Ck, où :

E désigne un degré d'efficacité désiré ;
XDI désigne la durée de mémoire immunitaire adéquate ;
XCV désigne une moyenne de Xj ;
Ck désigne une constante représentant un effet aléatoire pour tenir compte de la variation entre les échantillons cliniques et est obtenue par régression logique ; et
$\beta$0, $\beta$1, $\beta$2 sont des constantes obtenues par régression logique.

4. Méthode suivant la revendication 3, dans laquelle un modèle de régression logique pour obtenir les valeurs de Ck, $\beta_0$, $\beta_1$ et $\beta_2$ est sous la forme logit (p) = $\beta_0 + \beta_1$ Xi + $\beta_2$ Xj + Ck ; dans laquelle :

logit(p) est un vecteur représentant les statuts immunitaires des animaux;
Ck représente le milieu clinique dans lequel chaque animal a été choisi.

5. Méthode suivant la revendication 1, dans laquelle :

(a) l'animal est un chat ; et
(b) les chats sont considérés comme étant des chats à haut risque ou à risque bas d'après des questionnaires d'exposition à d'autres chats ou vecteurs de la maladie.

6. Méthode suivant la revendication 5, dans laquelle :

(a) les animaux à haut risque dans ces zones sont considérés comme étant immuns si aucun signe clinique de la maladie n'est observé,
(b) tous les autres animaux sont considérés comme étant immuns si :

(i) les titres de Ab déterminés par neutralisation sérique sont :

$\geq$ 1:16 pour le virus de la panleucopénie féline,
$\geq$ 1:2 pour l'herpèsvirus félin,
$\geq$ 1:4 pour le calicivirus félin,

ou
(ii) les titres Ab déterminés par agglutination au microscope sont :

$\geq$ 1:8 pour le virus Chlamydia psittaci félin,

(c) lorsque 80 % des animaux ne sont plus immunisés vis-à-vis dudit agent pathologique, ce point de temps est utilisé comme temps de fin d'immunité pour l'analyse de régression logique (équation de prévision de durée de mémoire immunitaire adéquate) de la durée de mémoire immunitaire adéquate.

7. Méthode suivant la revendication 1, dans laquelle :

(a) ledit animal est un chien ; et
(b) lesdits chiens sont choisis dans divers milieux cliniques vétérinaires dans des régions géographiques distinctes. Les chiens choisis ont été vaccinés au moins deux fois avec le vaccin Vanguard ou un vaccin concurrent comprenant les mêmes antigènes,
(c) lesdits chiens auront reçu une dose de rappel du vaccin Vanguard à environ 12 (9-14) mois,
(d) ledit milieu clinique aura conservé des bilans adéquats pour chaque chien, les bilans établissant la date de vaccination initiale et de n'importe quel cas de maladies et le diagnostic associé,
(e) lors du choix d'une pluralité d'animaux d'étude dans ou un plusieurs milieux cliniques, pour la participation, un questionnaire est déposé par le propriétaire du chien, décrivant le mode de vie du chien (c'est-à-dire haut à bas risque de maladie),
(f) lesdits chiens sont attribués à une première catégorie de risques définie par la fréquence d'exposition à d'autres chiens ou à des vecteurs de la maladie,
(g) lors de ladite participation, du sang est prélevé, ce qui représente le premier échantillon de sang, et les titres en anticorps sont déterminés pour tous les chiens pour les antigènes vaccinaux dans le vaccin Vanguard (CDV, CAV-2, CPI, CPV, Leptospira canicola et icterohaemorrhagiae),

(h) après prélèvement dudit échantillon de sang, le chien est revacciné (rappel) et 3 à 7 jours plus tard est resaigné pour prélever un second échantillon de sang,

(i) ledit second échantillon de sang est analysé d'une manière identique à l'analyse du premier échantillon de sang pour quantifier les taux d'anticorps,

(j) le statut immunitaire est déterminé par n'importe lequel des moyens suivants :

(i) les animaux classés en tant que n'importe quels animaux au-delà d'un faible risque d'exposition à la maladie (c'est-à-dire dans des catégories supérieures aux catégories à faible risque) et qui sont restés sains sont considérés comme étant immuns, c'est-à-dire sont des seconds animaux indicateurs,

(ii) les animaux qui sont classés dans les catégories à risque faible ou nul de la maladie et qui ont conservé des titres résiduels d'anticorps (voir ci-dessous) ou qui ont répondu au rappel dans les 3 à 7 jours et qui n'ont aucun antécédent de la maladie (voir ci-dessous) sont considérés comme étant immuns, c'est-à-dire sont des seconds animaux indicateurs,

(iii) les taux d'anticorps qui indiquent une immunité en cours, c'est-à-dire des seconds animaux indicateurs, sont également décrits de la manière suivante :

(1)
(2) CDV SN >/= 1:16
(3) CPV SN >/= 1:16
(4) CAV-1 SN >/= 1:16
(5) CAV-2 SN >/= 1:16
(6) CPI SN >/= 1:4
(7) Lepto. ictero >/= 1:8
(8) Lepto canicol >/= 1:8

(k) une régression logique est utilisée pour déterminer la durée de l'immunité en établissant le rapport entre le temps de vaccination et le statut immun ou non immun.

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **SCHULTZ R.D.** *Veterinary Medicine,* 1998, vol. 93, 233-254
- **MACY D.W.** *J. Am. Vet. Med. Assoc.,* 1995, vol. 207, 421-425
- Report of the American Association of Feline Practitioners and Academy of Feline Medicine Advisory Panel on Feline Vaccines. *J. Am. Vet. Med. Assoc.,* 1998, vol. 212, 227-241
- **SMITH.** *J. Am. Vet. Med. Assoc.,* 1995, vol. 207, 421-425
- **HENDRICK et al.** *Cancer Research,* 1992, vol. 52, 5391-5394
- **DUVAL et al.** *Journal of Veterinary Internal Medicine,* 1996, vol. 10, 290-295
- **W. JEAN DODDS.** Veterinary Vaccines and Diagnostics. *Advances in Veterinary Medicine,* 1999, vol. 41, 718-719
- **WOOD PR et al.** *Veterinary Immunology & Immunopathology,* 1996, vol. 54, 33-44
- **ALLEN JE et al.** *Immunology Today,* 1997, vol. 18, 387-392
- **WE SAUNDERS.** Vaccines. 1994
- **OLSON et al.** *Am. J. Vet. Res.,* 1988, vol. 49, 1460-1466
- **SPRENT et al.** *Science,* 1994, vol. 265, 1395-1400
- **ALDERINK et al.** *J. Vet. Res.,* 1995, vol. 207, 1016-1018
- **SCHULTZ ; SMITH.** *Am. J. Vet. Res.,* 1995, vol. 207, 421-425
- **ALDERINK et al.** *J. Am. Vet. Med. Assoc.,* 1995, vol. 207, 1016-1018
- **DODDS.** *Proc. Am. Holistic Vet. Med. Assoc.,* 1995, 74-80
- **DODDS.** Vaccine Related issues In Complementary and Alternative Veterinary Medicine. 1997, 701-702
- **SCOTT et al.** *Feline Pract.,* 1997, vol. 25, 12-19
- **JOHNSON et al.** *Journal of the American Veterinary Medical Association,* 1985, vol. 186, 149-152
- **PEDERSEN.** *Feline Practice,* 1995, vol. 23, 7-20
- **HAFFER et al.** *Vaccine,* 1990, vol. 8, 12-16
- **HARTMAN et al.** *Veterinary Immunology and Immunopathology,* 1984, vol. 7, 245-254
- **TENNANT et al.** *Research in Veterinary Science,* 1991, vol. 51, 11-18
- **BEY.** *American Journal of Veterinary Research,* 1981, vol. 42, 1130-1132
- **MITZEL et al.** *American Journal of Veterinary Research,* 1977, vol. 38, 1361-1363
- **LUFF et al.** *Vet. Rec.,* 1987, vol. 120, 270-273
- **STEEL, R.G.D. ; TORRIE, J.H.** Principles and Procedures of Statistics: A Biometrical Approach. 1980
- **CANNON, R. M. ; ROE, R.T.** *Livestock Disease Surveys: A Field Manual for Veterinarians,* 1982
- **SCHULTZ.** *Vet. Med.,* March 1998, 223-254
- Veterinary Vaccines and Diagnostics. **SERGE MARTINOD.** Advances in Veterinary Medicine. 1999, vol. 41, 661
- Manual of Clinical Laboratory Immunology. 1997
- Manual of Clinical Microbiology. 1999
- Clinical Microbiology Procedures Handbook. 1992
- **S.FAINE.** Leptospira and Leptospirosis. 1994
- *Manual of Clinical Laboratory Immunology,* 1997
- Expert Committee on Rabies, 8th Report. *World Health Organization, Technical Report Series no. 824,* 1992
- **COOPER et al.** *Bulletin Mensuel de la Societe Veterinaire Pratique de France,* 1991, vol. 75 (13), 1-152
- **AUBY et al.** *Recueil de Médecine Vétéinaire,* 1974, vol. 150, 33-36
- **POLLOCK et al.** *Journal of the American Veterinary Medical Association,* 1979, vol. 180, 37-43
- **HARTMAN et al.** *Veterinary Immunology and Immunopathology,* 1984, vol. 7, 245-254
- **BEMIS DA.** Bordetella bronchiseptica in dogs: Bacteriological studies, pathogenesis, immune response and antibiotic treatment. *Ph.D. Thesis,* 1976
- **SCOTT et al.** *Journal of the American Veterinary Medical Association,* 1970, vol. 156, 439-453
- **JOHNSON et al.** *Journal of the American Veterinary Medical Association,* 1971, vol. 158, 876-884
- **TOMPKINS et al.** *Companion Animal Practice,* 1988, 15-26
- *Council Report Journal of the American Veterinary Medical Association,* 1989, vol. 195, 314-317
- **AGRESTI.** *Categorical Data Analysis,* 1990, 84-91

- **BEITLER et al.** *Biometrics,* 1985, vol. 41, 991-1000
- **DAVIDIAN et al.** *Biometrika,* 1993, vol. 80, 475-488
- **HARVILLE et al.** *Biometrics,* 1984, vol. 40, 393-408
- **HOSMER et al.** *Applied Logistic Regression; Longford Computational Statistics and Data Analysis,* 1989, vol. 17, 1-15
- **SERGE MARTINOD.** Veterinary Vaccines and Diagnostics. *Advances in Veterinary Medicine,* 1999, vol. 41, 635